# EUROPEAN PATENT APPLICATION

(11) **EP 1 609 797 A1**
(43) Date of publication of application: **28.12.2005**
(21) Application number: 04722978.6
(22) Date of filing: 24.03.2004
(51) Int. Cl.: C07H 15/04, A61K 31/7032, A61K 31/7135, A61K 51/04, A61K 51/06, A61P 3/14, A61P 9/10, A61P 17/00, A61P 19/10, A61P 29/00, A61P 35/00, A61P 35/04

(54) **COMPOUND HAVING AFFINITY WITH CALCIFIED TISSUE**

(30) Priority: 26.03.2003 JP 2003085919
(71) Applicant: NIHON MEDI-PHYSICS CO., LTD., Nishinomiya-shi, Hyogo 662-0918 (JP)
(72) Inventor: KANAZASHI, Nobuhiko c/o NIHON MEDI-PHYSICS CO. LTD, Chiba 2990266 (JP); MORISHITA, Aki c/o NIHON MEDI-PHYSICS CO. LTD., Chiba 2990266 (JP); ITO, Osamu c/o NIHON MEDI-PHYSICS CO. LTD., Chiba 2990266 (JP); KURAMI, Miki c/o NIHON MEDI-PHYSICS CO. LTD., Chiba 2990266 (JP)
(74) Representative: Pohlmann, Eckart
(86) International application number: PCT/JP2004/004019
(87) International publication number: WO 2004/085452

(57) **Abstract**

The present invention provides a compound excellent in accumulation on calcified tissues and ensuring rapid excretion into urine as well as rapid clearance from blood, useful as a diagnostic agent and a therapeutic agent, etc. The compound is a compound having affinity with a calcified tissue represented by the following formula: (AC)ₐ-MC-(LI)_{b}. In the formula, MC is a mother nucleus and represents a residue of a compound having a plurality of functional groups selected from the group consisting of an amino group, an amide group, a hydroxyl group, a thiol group, a thioether group, a sulfonyl group a phosphonyl group, an aldehyde group, a carboxyl group, a carbonyl group, a halogen, and a cyano group; AC is a group having affinity with a calcified tissue; LI is a ligand for binding to a metal atom; and a is an integer of 1 or more and b is 0 or an integer of 1 or more.

## Description

### Technical Field

The present invention relates to a compound having high affinity with a calcified tissue and exhibiting rapid excretion into urine, and use thereof as a diagnostic agent, a therapeutic agent and the like.

### Background Art

In recent years, skeletal scintigraphy by nuclear medical technique is becoming an important tool in diagnosing bone diseases of an initial stage. Imaging agents for bone scintigraphy are required to have high excretability into urine and rapid clearance from blood and tissues as well as high bone affinity, etc. so that the time from administering agents till performing imaging can be shortened and scintigrams of high quality can be obtained. Today, phosphate compounds labeled with a radioisotope are used. At first, an inorganic polyphosphate labeled with 99m-technetium was tried. However, the inorganic polyphosphate labeled with 99m-technetium was problematic in that it was hydrolyzed to monophosphate in an aqueous solution and accordingly the clearance from blood was low.

In order to solve this problem, Yano et al. reported stannous Tc-99m-ethane-1-hydroxy-1-diphosphonate (Tc-99m-HEDP) which is an organic diphosphonate labeled with 99m-technetium (J. Nucl. Med. 14, 73, (1973) and United States Patent No. 3,735,001 specification). Since this compound exhibits relatively rapid clearance from blood, bone scintigraphy examination can be conducted in a shorter time after administering the agent, and 99m-technetium-labeled phosphate compounds analogous to Tc-99m-HEDP, i.e., 99m-technetium labeled organic diphosphonate compounds such as methane diphosphonate (MDP) and hydroxymethane diphosphonate (HMDP) have been widely used to date. Although preparations for bone scintigraphy using these compounds accumulate at the site where osseous calcification proceeds and further shorten the time from administering the agent till performing imaging, they still take about three hours after administration, which cannot be said to be sufficiently short.

Generally, when a preparation for bone scintigraphy is slow in the clearance from blood and/or soft tissues and the excretion into urine, the time from administering the agent till performing imaging becomes longer in order to improve contrast of image. It is considered that polymer structure of technetium-bisphosphonate complexes is one of the factors that affect the clearance of technetium-labeled phosphate compounds. Although an attempt to promote the clearance at an early stage after administration by changing a bisphosphonate compound from a polymer structure to a monomolecular structure was made on a bisphosphonate compound labeled with 123-iodine (WO89/11877), but the efficacy is not necessarily sufficient.

From the same viewpoint, various organic phosphonic acid compounds such as bisphosphonic acid derivatives have also been proposed (Japanese Patent Laid-Open No. 59-205331, Japanese Patent Laid-Open No. 57-50928, Japanese Patent Laid-Open No. 63-500849, Japanese Patent Laid-Open 2001-114792), but they are still required to be improved in excretability in urine and blood clearance so that the time from administering the agent till taking an image can be shortened.

### Disclosure of the Invention

Therefore, one object of the present invention is to provide novel organic phosphonic acid derivatives which can be compounds with excellent affinity to calcified tissue but excreted into urine at high rate when they fail to accumulate in calcified tissue by designing the derivatives so that their organic phosphonic acid groups do not readily form complexes and the molecular size of the compounds with excellent affinity to calcified tissue is controlled, the other is to provide its use as a diagnostic agent, a therapeutic agent and the like.

The present inventors have conducted various studies on organic phosphonic acids and other compounds having affinity with a calcified tissue to attain the above-mentioned object, and have found that compounds which are represented by the following general formula: (AC)ₐ-MC-(LI)_{b} (wherein, MC is a mother nucleus, AC is a group having affinity with a calcified tissue, and LI is a ligand for binding to a metal atom; a is an integer of 1 or more; b is 0 or an integer of 1 or more) and have a mother nucleus MC of a controlled molecular size exhibit excellent affinity with a calcified tissue while the compounds that fail to accumulate in calcified tissues exhibit high excretability into urine, and thereby completed the present invention.

That is, according to one aspect of the present invention, a compound having affinity with a calcified tissue represented by the following general formula:

(AC)ₐ-MC-(LI)_{b}

(wherein MC is a mother nucleus and represents a residue of a compound having a plurality of functional groups selected from the group consisting of an amino group, an amide group, a hydroxyl group, a thiol group, a thioether group, a sulfonyl group, a phosphonyl group, an aldehyde group, a carboxyl group, a carbonyl group, a halogen, and a cyano group;
AC is a group having affinity with a calcified tissue;
LI is a ligand for binding to a metal atom; and
a and b are integers of 1 or more) is provided.

Here, the ligand LI can bind to a metal atom, and thus may form a complex with a metal atom but does not need to form a complex. Since the LI moiety plays a central role in complex formation ability, and thus the AC moiety does not readily form a complex compound, the present compounds exhibit a rapid clearance from the blood and/or soft tissue and also a rapid excretion into urine advantageously.

Preferable compounds of the present invention are represented by the formula: (AC)ₐ-MC-(LI)_{b}
(wherein MC represents a residue of a compound selected from the group consisting of a monosaccharide, an oligosaccharide, an amino oligosaccharide, a cyclodextrin and a saccharide dendrimer; a and b are integers of 1 or more). AC in the above formula is preferably a calcified tissue-affinity group which comprises a polyaspartic acid group, a polyglutamic acid group and an organic phosphonic acid group, and more preferably it is an organic phosphonic acid group.

More preferable compounds of the present invention are represented by the formula: (AC)ₐ-MC-(LI)_{b}
(wherein MC represents a residue of a compound selected from the group consisting of an oligosaccharide, an amino oligosaccharide, a cyclodextrin and a saccharide dendrimer, and the group AC having affinity with a calcified tissue is bonded to any one of the constituent monosaccharides of the mother nucleus MC, and the ligand LI for binding to a metal atom is bonded to a constituent monosaccharide other than the above-mentioned constituent monosaccharide; a and b are integers of 1 or more). A plurality of the calcified tissue-affinity group AC or the ligand LI for binding to a metal atom may be bonded to the mother nucleus MC.

Furthermore, according to another aspect of the present invention, a compound having affinity with a calcified tissue represented by the formula: (AC)ₐ-MC (wherein MC is a mother nucleus and represents a residue of a compound having a plurality of functional groups selected from the group consisting of an amino group, an amide group, a hydroxyl group, a thiol group, a thioether group, a sulfonyl group, a phosphonyl group, an aldehyde group, a carboxyl group, a carbonyl group, a halogen, and a cyano group;
AC is a group having affinity with a calcified tissue; and
a is an integer of 1 or more) is provided. This compound is advantageous in that it has a mother nucleus MC of a controlled molecular size and exhibits excellent affinity with a calcified tissue by virtue of the AC while the compound which fails to accumulate in calcified tissues exhibits high excretability in urine by virtue of the MC.

In the compound represented by the formula (AC)ₐ-MC, it is preferable that the MC is a residue of a compound selected from the group consisting of a monosaccharide, an oligosaccharide, an amino oligosaccharide, a cyclodextrin and a saccharide dendrimer and the AC is preferably a calcified tissues-affinity group comprising a polyaspartic acid group, a polyglutamic acid group and an organic phosphonic acid group, and the AC is more preferably an organic phosphonic acid group.

According to a preferable embodiment, the compound of the present invention comprises a metal atom or an isotope of a halogen atom, carbon, oxygen, nitrogen, sulfur or phosphorus in at least one of the mother nucleus MC, the calcified tissue-affinity group AC and the ligand LI. This embodiment is particularly preferable for use as a diagnostic agent.

Japanese Patent Laid-open No. 10-501218 discloses a 99m-technetium mono-, di- or polyphosphonate complex composition which has a composition variable with conditions of heating by autoclaves, microwaves and the like. This is an attempt to improve aggravation of the clearance caused by the formation of a polymer structure of the radioactive metal labeled phosphate compound. In this method, however, co-existence of polyphosphonates having polymer structures is still not avoided.

On the other hand, the compound represented by the formula (AC)ₐ-MC-(LI)_{b} of the present invention is characterized in that the labeling function by a radioactive nuclide is assigned to the ligand LI, and the opportunity for bisphosphonic acid to participate in complex formation is reduced, thereby contemplating a more advantageous improvement of accumulation to the bone. The same technical idea is apparently applicable to the calcified tissue-affinity group AC other than the bisphosphonic acid. The difference in the complex formation ability between the ligand LI and the calcified tissue-affinity group AC can be proved by using an analog of the compound of the present invention. The idea that a calcified tissue-affinity group AC forms no complex can be proved by selecting labeling conditions such as radioactive metal nuclides, concentration, pH and reducing agents. For example, it can be proved by a coexistence labeling method using DTPA or MAG3 together with HMDP.

In addition, the compound represented by the formula (AC)ₐ-MC according to the present invention has affinity with a calcified tissue in itself, and thus is useful not only as a therapeutic agent but also as a diagnostic agent since it can be labeled by allowing an isotope of a metal atom or a halogen atom, carbon, oxygen, nitrogen, sulfur or phosphorus to be contained in at least one of the mother nucleus MC and the calcified tissue-affinity group AC.

The use of diagnostic agents in diagnostic medicine has been rapidly increasing. Conventionally a composition or a substance labeled with a radioisotope has been administered to a living body in nuclear medicine diagnosis, wherein the radiation that the composition or substance emits is detected by a scintillation camera and the distribution and behaviour of the composition or substance in the living body is expressed non-invasively as an image, and this technique has been used for early detection of various diseases and elucidation of pathologic conditions. The composition and substance labeled with a radioisotope are called radioactive imaging agents, and those suitable for specific purposes have been developed. Furthermore, in MRI diagnosis, contrast of tissues can be enhanced by administering a composition or substance containing a paramagnetic metal species which increases the relaxation of surrounding protons.

On the other hand, referring to therapeutic agents, it has been known that bisphosphonic acids have, by virtue of their affinity with calcified tissues, an effect of inhibiting bone absorption and increase of serum calcium level resulting from enhancement of the bone absorption. Thus, they have been introduced to clinical practice as active substances in drugs for treating diseases which morbid condition is considered to have a significant relation with bone absorption, for example, Paget's disease, hypercalcemia, bone metastasis of cancer and osteoporosis. In addition, their pharmacological actions, for example, prevention of aggravation after cancer metastasis, relief of bone pain, prevention of periodontal diseases, etc. are known.

Furthermore, due to the affinity with calcified tissues, the compound of the present invention can be used in diagnosis and medical treatment of not only bone diseases but also calcified lesions of blood vessels, such as arteriosclerosis at calcified blood vessel sites or the like, and chronic inflammatory diseases.

Accordingly, since the compound of the present invention selectively accumulates in calcified tissues in general in a living body and is rapidly excreted in urine, it is useful as a diagnostic agent or a therapeutic agent of various diseases mentioned above.

Specifically, the compound of the present invention suitably subjected to radioactive nuclide labeling is useful as an active ingredient for diagnosing bone diseases such as bone metastasis, osteoporosis, Paget's disease, bone fracture, heterotopic ossification, bone formation or bone dissolution and for diagnosing calcified blood vessel sites including arteriosclerosis. When it is applied to visualization of the skeletal scintigraphy for finding out affected sites, the compound of the present invention is intravenously administered to a body of mammals including humans, and, subsequently radioactivity distribution in the body is measured. The radioactivity distribution is measured by using a commonly known equipment (gamma camera, etc.).

In addition, the compound of the present invention is applicable as therapeutic agents for inflammatory bone diseases such as rheumatoid arthritis and lumbago and for pain relieving, and as anticancer drugs for bone cancer and its metastasis, prevention of bone metastasis of cancer, and the like. Furthermore, the compound of the present invention can be used in diagnosis for selection of a therapeutic agent and also for pharmacometrics such as efficacy estimation.

The compound of the present invention can be used as an imaging agent in various types of diagnostic imaging using radiation, nuclear magnetic resonance, X-ray, ultrasonic wave, etc., or a therapeutic agent depending on the type of labeling substance contained therein. The compound of the present invention or a salt thereof can be provided as a pharmaceutical composition with at least one pharmaceutically acceptable carrier.

### Best Mode for Carrying Out the Invention

### (1) Mother nucleus MC

In the present invention, the mother nucleus MC is not limited as long as it has a plurality of functional groups available for chemically bonding to the group AC having affinity with a calcified tissue and the ligand LI. Specifically, a residue of a compound is included which has a plurality of functional groups selected from the group consisting of an amino group, an amide group, a hydroxyl group, a thiol group, a thioether group, a sulfonyl group, a phosphonyl group, an aldehyde group, a carboxyl group, a carbonyl group, a halogen and a cyano group.

Therefore, the mother nucleus MC may be a monosaccharide, oligosaccharide, polysaccharide, amino acid, oligopeptide, polypeptide, nucleotide, oligonucleotide, poly nucleotide, protein, protein fragment, chemical derivatives thereof, or a synthetic polymer, as long as it has a plurality of the above-mentioned functional groups.

The molecule size of the compound of the present invention can be controlled by the size of the mother nucleus MC, and therefore, transfer of the compound between blood vessel and tissues can be controlled by changing the molecule size thereof depending on the specific use so that the compound can effectively pass through capillary vessel pores (5 to 10 µm) and selectively act on the target tissue.

Preferably, the mother nucleus MC is a residue of a saccharide compound selected from the group consisting of a monosaccharide, an oligosaccharide, an amino oligosaccharide, a cyclodextrin and a saccharide dendrimer, more preferably, a residue of an oligosaccharide, an amino oligosaccharide, a cyclodextrin, a saccharide dendrimer, and particularly preferably a residue of an oligosaccharide and an amino oligosaccharide.

The monosaccharide includes glucose, mannose, galactose, glucosamine, mannosamine, galactosamine, etc.

The oligosaccharide includes those containing as a constituent monosaccharide one or more selected from the group consisting of glucose, mannose, galactose, etc. These oligosaccharides may be linear or branched, and it is preferable from a viewpoint of the transferability of the compound of the present invention between the blood vessel and tissues that the oligosaccharide is a polymer of 2 to 20 saccharide units. The oligosaccharide may be those formed of constituent monosaccharides that are mutually α- or β-linked, or those formed of constituent monosaccharides that are mutually 1-3, 1-4 or 1-6-linked. Specific examples of preferable oligosaccharides include maltotriose, maltotetraose, maltopentaose, maltohexaose, maltoheptaose, isomaltotriose, isomaltotetraose, isomaltopentaose, isomaltohexaose, isomaltoheptaose, cellotriose, cellotetraose, cellopentaose, cellohexaose, laminaritriose, laminaritetraose, laminaripentaose, laminarihexaose, laminariheptaose, erlose, panose, raffinose, etc. The oligosaccharide may be those reduced in some parts such as at a terminal end or those not reduced, but those reduced are preferable. Dialdehyde-saccharides thereof are also included in the oligosaccharide.

The amino oligosaccharide includes those containing as a constituent monosaccharide one or more aminosaccharide selected from the group consisting of glucosamine, mannosamine, galactosamine, etc. These may be linear or branched, and it is preferable from a viewpoint of the transferability of the compound of the present invention between the blood vessel and tissues that the amino oligosaccharide is a polymer of 2 to 20 saccharide units and a polymer of 2 to 13 saccharide units is more preferable. The amino oligosaccharide may be those formed of constituent monosaccharides that are mutually α- or β-linked, or those formed of constituent monosaccharides that are mutually 1-3, 1-4 or 1-6-linked. Chitosan oligosaccharides having 2 to 10 repeting units of constituent monosaccharide or galactosamine oligosaccharides having 2 to 10 repeting units of constituent monosaccharide can be used as particularly preferable amino oligosaccharides, and specifically chitosan oligosaccharides such as chitosan dimer, chitosan trimer, chitosan tetramer, chitosan pentamer and chitosan hexamer and galactosamine oligosaccharides such as galactosamine dimer, galactosamine trimer, galactosamine tetramer, galactosamine pentamer and galactosamine hexamer can be exemplified. The amino oligosaccharide may be those reduced in some parts such as at a terminal end or those not reduced, but those reduced are preferable. In addition, amino oligosaccharides in which a part of the amino groups is N-acetylated and dialdehyde saccharides thereof are also included in the amino oligosaccharide.

The cyclodextrin includes α-, β- and γ-cyclodextrins. Dialdehyde saccharides in which the positions 2 and 3 of the constituent monosaccharide are reduced are also included in the cyclodextrin.

The saccharide dendrimer includes, for example, a linear or branched saccharide bonded to a core consisting of a polycarboxylic acid or alkyl polycarboxylic acid. The structure of the saccharide dendrimer is expressed by generation which means a structure where circles can be drawn from the inner core to the outside, and the number of the circles of polycarboxylic acid is preferably 1 to 5 generations, more preferably 1 to 3.

Another saccharide dendrimer includes a linear or branched saccharide bonded to a core consisting of a polyamine or alkylpolyamine. This saccharide dendrimer takes a structure where circles can be drawn from the inner core to the outside on the basis of the constituting nitrogen atoms, and saccharides are bonded to the outermost nitrogen atoms to constitute the saccharide dendrimer. In this case, the number of the nitrogen circles is preferably 1 to 5 generations, more preferably 1 to 3.

In addition, the mother nucleus MC which has a phosphonyl group includes inositol trisphosphates, etc., and the mother nucleus MC which has a sulfonyl group includes chondroitin sulfates, heparan sulfates, keratan sulfates, etc. The mother nucleus MC which has a carboxyl group or a carbonyl group includes glucuronic acid, etc., and the mother nucleus MC which has a halogen includes acetobromo-α-D-glucuronic acid methylester, etc., and the mother nucleus MC which has a cyano group includes cyanomethylmannose, etc.

### (2) Group AC having affinity with a calcified tissue

The group AC having affinity with a calcified tissue is not restricted as long as the compound has affinity with a calcified tissue found in bones, blood vessels, etc., and typically includes polyaspartic acid, polyglutamic acid, organic phosphonic acid and derivatives thereof.

The organic phosphonic acid which constitutes the group AC having affinity with a calcified tissue includes, for example, a residue of a diphosphonic acid represented by the following formula I, derivatives thereof, and salts thereof.
(wherein, 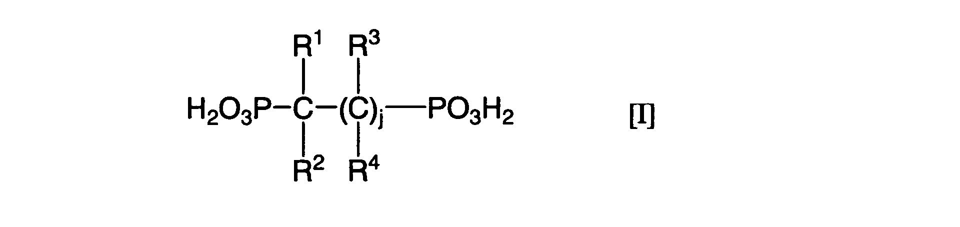 R¹ and R³, which are the same or different, each represents a formula -(CR⁵R⁶)ₖ-R⁷₁ - (CR⁸R⁹)ₘ-R¹⁰ₙ-(CR¹¹R¹²)ₒ-R¹³ₚ-(CR¹⁴R¹⁵)_{q}R¹⁶ (wherein R⁵, R⁶, R⁸, R⁹, R¹¹, R¹², R¹⁴, R¹⁵ and R¹⁶ are groups each independently selected from the group consisting of H, -OH, -COOH, -C(NH₂)=NH, -CN, -SO₃H, -NR¹⁷₂ and a halogen atom, R¹⁷ is independently H or - (CH₂)ᵣCH₃ respectively, R⁷, R¹⁰ and R¹³ are groups each independently selected from the group consisting of sulfur, oxygen, amide, imide, a divalent heterocycle consisting of 3 to 12 atoms and a cyclic hydrocarbon (Ar-(R¹⁸ᵣ-R¹⁹)ₛ), R¹⁸ is -CR⁵R¹⁷, R¹⁹ is independently selected from the group consisting of H, -OH, -COOH, -C(NH₂)=NH, - CN, -SO₃H, -NH₂, -NHMe, -NMe₂ and a halogen atom; k, 1, m, n, o, p and q are each independently 0 or an integer of 1 or more, r is 0 to 3, s is 0 to 12, and the sum total of k, 1, m, n, o, p and q is 0 to 12);
R² is a group selected from H, -OH, -NH₂, -NHMe, -NMe₂, - CN, and a lower alkyl group (which may be substituted with one or a plurality of polar groups);
R⁴ is a group selected from H, -OH, -NH₂, -NHMe, -NMe₂, - CN, -SO₃H, a halogen and a lower alkyl group (which may be substituted with one or a plurality of polar groups); and
j is 0 or 1 (provided that, when j is 0, R¹ is not be H and when j is 1, both of R¹ and R³ cannot be H).

The organic phosphonic acid represented by the above-mentioned formula I includes, for example, ethylene glycol-1,2-bisphosphonic acid, diphosphonomethanesulfonic acid, 2,2-diphosphonoethane-sulfonic acid, 2,2- diphosphono-2-hydroxyethane-sulfonic acid, 1,1- diphosphono-2-hydroxyethane-sulfonic acid, N,N-dimethyl-1-aminoethane-1,1-diphosphonic acid, the derivatives thereof, etc.

As an organic phosphonic acid represented by the above-mentioned formula I, α-geminal-bisphosphonic acid, i.e., the bisphosphonic acid in which j is 0 and which has P-C-P bond or derivatives thereof can be used preferably. In this case, R¹ and R² which are the substituent groups on the alpha carbon may be hydrogen, a hydroxyl group, an amino group, a halogen group, a carboxylic acid group, a sulfonic acid group, a lower alkyl group, a lower alkyl alcoholic group, a cyano group, etc., and either R¹ or R² bonds to the functional group of the mother nucleus MC.

That is, among the compounds represented by the above-mentioned formula I, compounds in which j is 0 (therefore, R¹ is not H) and R¹ is represented by the formula -(CR¹⁴R¹⁵)_{q}R¹⁶ (wherein R¹⁴, R¹⁵ and R¹⁶ are groups each independently selected from the group consisting of H, -OH, -COOH, -C(NH₂)=NH, -CN, -SO₃H, -NR¹⁷₂ and a halogen atom; R¹⁷ is independently H or -(CH₂)ᵣCH₃; respectively; q is 0 or an integer of 1 to 9; and r is 0 to 3) or the formula -R¹³ₚ-R¹⁶ (wherein R¹⁶ is a group selected from the group consisting of H, -OH, -COOH, - C(NH₂)=NH, -CN, -SO₃H, -NR¹⁷₂ and a halogen atom; R¹⁷ is independently H or -(CH₂)ᵣCH₃, r is 0 to 3; R¹³ is a group represented by thiophenylene, pyrimidinylene, pyrrolidinylene, phenylene, ether, or thioether) and R² is H or -OH are preferable.

Among the compounds represented by the above-mentioned formula I, compounds in which j is 0 (therefore, R¹ is not H) and R¹ is represented by the formula - (CR¹⁴R¹⁵)_{q}R¹⁶ (wherein R¹⁴ and R¹⁵ are H, R¹⁶ is a group each independently selected from the group consisting of H, - OH, -COOH, -C(NH₂)=NH, -CN, -SO₃H, -NR¹⁷₂ and a halogen atom; R¹⁷ is independently H or - (CH₂)ᵣCH₃; r is 0 to 3; q is 0 or an integer of 1 to 9, preferably 0 or an integer of 1 to 5) and R² is H or -OH are more preferable.

Such bisphosphonic acids include, for example, methane diphosphonic acid (MDP), hydroxymethane diphosphonic acid (HMDP), 1-hydroxyethane-1,1-bisphosphonic acid (EHDP), dimethylamino methylene diphosphonic acid (DMAD), 3,3-diphosphono-1,2-propane diphosphonic acid (DPD), Tildronate, etc., and an ester or salt thereof can also be used. The structural formulae of these compounds are as follows:

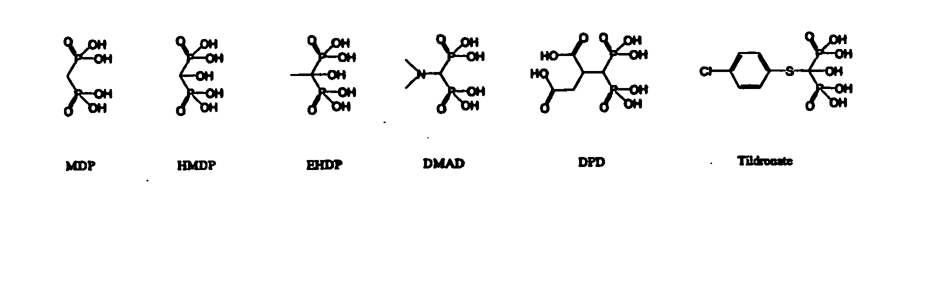

Methane diphosphonic acid (MDP) etc. can be used as an organic phosphonic acid of the present invention by deriving as shown below according to a known method (for example, a method described in J. Org. Chem., 66 (11), 3704-3708, (2001)).

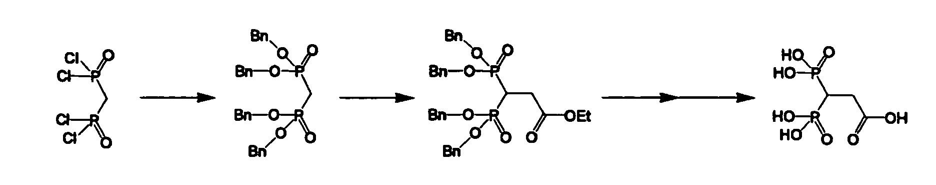

Alendronate which is a hydroxybisphosphonic acid can be used as an organic phosphonic acid of the present invention according to a known method (for example, a method described in Heteroatom Chemistry, 11 (7), 556-561 (2000)) as shown below.

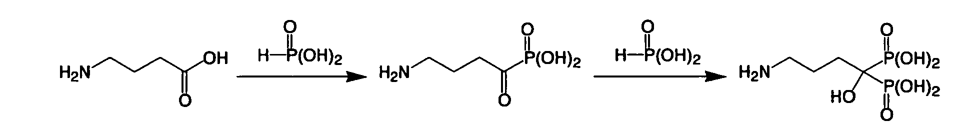

The method of chemically bonding the above-mentioned organic phosphonic acid to the mother nucleus MC includes amidation, esterification, imidation, etc., for example.

Furthermore, as organic phosphonic acids, organic aminophosphonic acid derivatives in which the group represented by the formula II is bonded to an amine nitrogen atom, an ester or salt thereof can also be used. 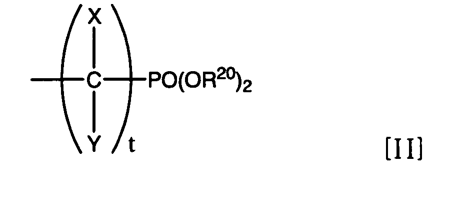 (wherein t is an integer of 1 to 8; X and Y are each independently selected from hydrogen, a halogen group, a hydroxyl group, a carboxyl group, a carbonyl group, a phosphonic acid group, and a hydrocarbon group having 1 to 8 carbon atoms, and when t is larger than 1, each X and Y may be the same or different; R²⁰ is selected from hydrogen, a silyl group, an alkyl group, a benzyl group, sodium and potassium).

Another example of the organic phosphonic acid includes phosphonic acid derivatives represented by the formula III, esters or salts thereof. 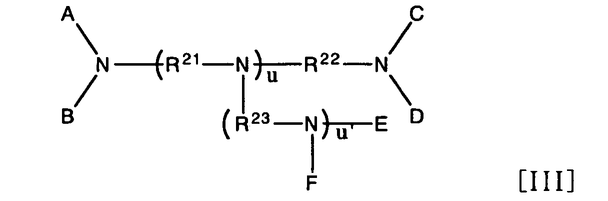 (wherein each u and u' is independently an integer of 0 to 5, and preferably is 0, 1, or 2; R²¹, R²² and R²³ are each independently -(CH₂)ᵥ- (v= 1 to 5); A, B, C, D, E, and F are each independently selected from the group consisting of hydrogen, a methyl group, an ethyl group, an isopropyl group, a pivaloyl group, a benzyl group, an acetyl group, a trifluoroacetyl group, and groups of the following formulae IV-1 to 3, and one of A, B, C, D, E and F is the group of following formula IV-1. 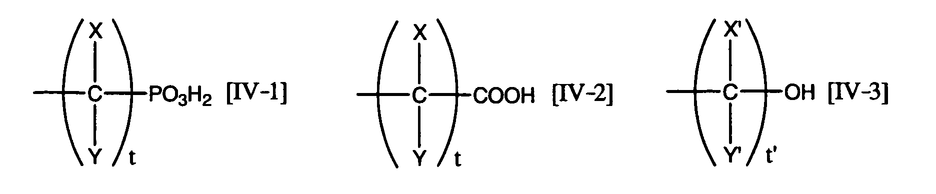 (wherein t, X and Y are the same as in the above-mentioned formula II; t' is 2 or 3; X' and Y' are each independently selected from hydrogen, a methyl group and an ethyl group, and each X' and Y' may be the same or different)).

Examples of the phosphonic acid derivative represented by the formula III include ethylenediaminetetramethyleneprosphonic acid (EDTMP), diethylenetriaminepentamethylenephosphonic acid (DTPMP), hydroxyethylethylenediaminetrimethylenephosphonic acid (HEEDTMP), nitrilotrimethylenephosphonic acid (NTMP), tris(2-aminoethyl) aminehexamethylenephosphonic acid (TTHMP), 1-carboxyethylenediaminetetramethylenephosphonic acid (CEDTMP), bis(aminoethylpiperazine) tetramethylenephosphonic acid (AEPTMP), N-methylethylenediaminetrimethylenephosphonic acid (MEDTMP), N-isopropylethylenediaminetrimethylenephosphonic acid (IEDTMP), N-benzylethylenediaminetrimethylenephosphonic acid (BzEDTMP), etc.

Other organic phosphonic acids which can be used include polyvalent phosphoric acid derivatives typically exemplified by EDTMP, DTPMP, etc. Similarly to bisphosphonic acids, these compounds have affinity with calcified tissues such as bone, and are considered promising as therapeutic agents.

Among the polyaspartic acids, those of polymerization degree of 4 to 12 can be used preferably. Among the polyglutamic acids, those of polymerization degree of 4 to 12 can be used preferably.

The method of chemically bonding the above-mentioned organic phosphonic acid, polyaspartic acid, or polyglutamic acid to the mother nucleus MC includes amidation, esterification, imidation, etc., for example.

### (3) Ligand (LI)

The ligand (LI) which is capable of binding to a metal atom in the compound of the present invention includes, for example, the ligand which can form a complex with a metal atom or a metal ion. The ligand (LI) as used herein means a compound which can form a stable complex with a metal atom or a metal ion.

Typical examples of the ligand (LI) include polydentate or multidentate ligands i.e., ligands which contain a plurality of coordinating atoms per one ligand molecule. The coordinating atom as used herein is defined as an atom having a free electron pair which can bind to a metal atom. This molecule has preferably a plurality of coordinating atoms. The coordinating atom is selected from nitrogen, oxygen, sulfur, phosphorus and carbon, and nitrogen and/or oxygen and/or sulfur are suitable coordinating atoms.

Typical examples of the polydentate or multidentate ligand include chain or cyclic polyaminopolycarboxylic acid or chain or cyclic polyaminopolyphosphonic acid, or derivatives thereof. Specific examples of the polyaminopolycarboxylic acid include ethylenediaminediacetic acid, nitrilotriacetic acid, ethylenediaminetetraacetic acid (hereinafter abbreviated as EDTA), diaminocyclohexanetetraacetic acid, diethylenetriaminepentaacetic acid (DTPA), triethylenetetraminehexaacetic acid (TTHA), 1,4,7,10-tetraazacyclododecane-1,4,7,10-tetraacetic acid (DOTA), 1,4,8,11-tetraazacyclotetradecane-1,4,8,11-tetraacetic acid (TETA) and derivatives thereof. Specific example of polyaminopolyphosphonic acid includes EDTMP and derivatives thereof.

The derivatives of polyaminopolycarboxylic acids include, for example, compounds in which one or a plurality of carboxyl groups of polyaminopolycarboxylic acids are subjected to esterification, halogenation, addition of protection group, or replacement with a hydrocarbon group which has a substituent group other than carboxylic acid; compounds in which a hydrocarbon group having a substituent group other than carboxylic acid or a substituent group having no hydrocarbon group is introduced into a hydrocarbon moiety which constitutes the polyaminopolycarboxylic acid; and compounds in which an ether group etc. is introduced into a carbon backbone moiety of the polyaminopolycarboxylic acid. Specifically, hydroxyethylethylenediamine triacetic acid, diaminopropanoltetraacetic acid, N,N-bis(2-hydroxybenzyl)ethylenediaminediacetic acid, glycol ether diaminetetraacetic acid, etc. can be mentioned.

In addition to these, those selected from the group consisting of cyclam, N{1-2,3-dioleyloxy)propyl}-N,N,N-triethylammonium (DOTMA), mercaptoacetylglycylglycylglycine (MAG3), ethylene cysteine dimer (ECD), hydrazinonicotinyl (HYNIC), lysine-tyrosine-cysteine (KYC), cysteine-glycine-cysteine (CYC), N,N'-bis(mercaptoacetamido)ethylenediamine (DADS), N,N'-bis(mercaptoacetamido)-2,3-diamine propanoic acid (CO₂DADS) (European Patent No. 0173424 , US Patent No. 4673562 specification), N,N'-bis(2-mercaptoethyl)ethylenediamine (BATs) (European Patent No. 0163119 and No. 0200211), thiosemicarbazone, propylene amineoxime (PnAO), other amineoxime ligand (European Patent No. 0123504 and No. 0194843) and derivatives thereof can be exemplified as ligand (LI).

In addition to these, as a ligand (LI), a group of multidentate ligand containing sulfur and nitrogen as coordinating atoms such as 6-hydrazinonicotinic acid, diaminodithiol, monoaminomonoamidodithiol, diamidodithiol and triamidothiol can be also used for a complex formation part. Referring to chelating groups, for example, diaminodithiol includes N,N'-bis(2-mercaptoethyl)ethylenediamine, 2,2,9,9-tetramethyl-4,7-diaza-1,10-decanethiol; monoamidemonoaminodithiol includes N-2-mercaptoethyl-2-mercaptoethylaminoacetamide and N-(2-mercaptoethyl)aminoethyl-2-mercaptoacetamide; diamidodithiol includes 1,2-ethylene bis(2-mercaptoacetamide); and triamidothiol includes mercaptoacetylglycylglycylglycine.

The multidentate ligand further includes huge 4-,5- ,6-,7- and 8-dentate compounds containing nitrogen which are cyclic or open-chain and may or may not have other coordinating atoms or unsaturated bonds.

Bonding of these ligands (LI) and the mother nucleus MC can be performed by chemically bonding functional groups of the ligands which are not important for forming a complex with metal and the functional group of the mother nucleus MC mutually.

The ligand (LI) may be a bifunctional ligand. The bifunctional ligand is a compound that has in molecule a site for binding to the mother nucleus MC and a site for forming a complex with a metal. Therefore, by way of the functional groups which exist in the mother nucleus MC and are available for binding to the bifunctional ligand, the bifunctional ligands to the number corresponding to the number of the functional groups can be chemically bonded to the physiologically acceptable substances.

The site for forming a complex with a metal is not limited to specific types as long as it is a multidentate ligand which forms a stable complex with a respective metal, and it can be typically selected from cyclic or chain polyaminopolycarboxylic acid or cyclic or chain polyaminopolyphosphonic acid as mentioned above and, for example, EDTA, DTPA, DOTA, TETA or derivatives thereof, or EDTMP or derivatives thereof, or MAG3, cyclam, BAT, ECD, DADS and PnAO or derivatives thereof can be used.

As a reactive coupling group in the bifunctional ligand which constitutes a site for binding to the mother nucleus MC, not only normally-employed amino group, carboxyl group and thiol group, but also active halogen, alkoxy ester, N-hydroxysuccinimide ester, imide ester, maleimide, thiophthalimide, isothiocyanate, acid anhydride, etc. can be specifically exemplified.

Specific examples of the bifunctional ligand include compounds having polyaminopolycarboxylic acid or polyaminopolyphosphonic acid as a site for forming a complex with a metal, for example, 1-(p-isothiocyanatebenzyl)-DTPA [Martin WB et al., Inorg. Chem., 25, p. 2772-2781 (1986)], anhydrous DTPA, 2-(p-isothiocyanatebenzyl)-1,4,7,10-tetraazacyclododecane-1,4,7,10-tetraacetic acid [U.S. Patent No. 4678667 specification], succinimidyl-6-hydrazinonicotinate [Abrams, M.J. et al., J. Nucl. Med., 31, p.2022-2028 (1990)], DTPA-mono(2-aminoethylamide), DTPA-mono(3-aminopropylamide), DTPA-mono(6-aminohexylamide) [Japanese Patent No. 2815615], 1-(4-aminobenzyl)-EDTA, 1-(4-isothiocyanobenzyl)-EDTA, 1-[4-(3-maleimide propyl)amidebenzyl-EDTA, 1-[4-(5-maleimidepentyl)amidobenzyl]-EDTA, etc.

The bonding of the mother nucleus MC and a bifunctional ligand can be performed by a method known per se. For example, the reaction of the mother nucleus MC and a bifunctional ligand having, as a reactive coupling group, acid anhydride [Hnatowich DJ et al., Int. J. Appl. Rad. Isot., 33, p. 327-332 (1982)], isothiocyanate [Esteban JM et al., J. Nucl. Med., 28, p. 861-870 (1987)], alkoxyester [Washburn LC et al., Nucl. Med. Biol., 18, p. 313-321(1991)] or active halogen [Fourie PJ et al., Eur. J. Nucl. Med., 4, p. 445-448 (1979)] can be performed according to the description of the known literatures cited above.

A preferable ligand (LI) is selected from the group consisting of ethylenediaminetetraacetic acid (EDTA), diethylenetriaminepentaacetic acid (DTPA), triethylenetetraminehexaacetic acid (TTHA), cyclam, 1,4,8,11-tetraazacyclotetradecane-1,4,8,11-tetraacetic acid (TETA), 1,4,7,10-tetraazacyclododecane-1,4,7,10-tetraacetic acid (DOTA), N{1-2,3-dioleyloxy)propyl}-N,N,N-triethylammonium (DOTMA), mercaptoacetylglycylglycine (MAG3), ethylene cysteine dimer (ECD), hydrazinonicotinyl (HYNIC), lysine-tyrosine-cysteine (KYC), cysteine-glycine-cysteine (CYC), N,N'-bis(mercaptoacetamido)ethylenediamine (DADS), N,N'-bis(mercaptoacetamido)-2,3-diamine propanoic acid (CO2DADS), N,N'-bis(2-mercaptoethyl)ethylenediamine (BATs), thiosemicarbazone, propylene amineoxime (PnAO), and other amineoxime ligand and derivatives thereof.

Complexation of the metal atom to the above-mentioned ligand (LI) can be performed by ordinary methods. The metal atom for complexation can be suitably selected according to use, and a metal atom useful as a labeling substance can be usually selected. The metal atom includes a metal atom having radioactivity, paramagnetism or X-ray impermeability or an ion thereof.

The compound of the present invention can include a metal atom or an isotope of halogen atom, carbon, oxygen, nitrogen, sulfur or phosphorus in at least one of the mother nucleus MC and the group AC having affinity with a calcified tissue, irrespective of the existence of ligand LI. As a halogen atom, fluorine, bromine, iodine, etc. can be used preferably. Introduction of these halogen atoms can be performed by introducing a substituent group containing a leaving group such as a tosyl group into the mother nucleus MC or the group AC having affinity with a calcified tissue, and replacing this substituent group with a halogen atom. It can be also performed by introducing a substituent group such as trialkyl tin represented by the formula Sn(R₃) and other metal alkyl groups into the mother nucleus MC or the group AC having affinity with a calcified tissue, and replacing this substituent group with a halogen atom. A methyl group, an ethyl group, a propyl group, a butyl group, etc. can be used as the alkyl group of the metal alkyl group. A preferable metal alkyl group is trimethyltin or tributyltin.

Alternatively, the isotope elements, for example, 11-C can be introduced by reacting methyl iodide (¹¹CH₃I) with the amino group of glucosamine of the mother nucleus MC (yielding -NH¹¹CH₃) or by reacting a carboxylic acid of polycarboxylic acid (DTPA etc.) which constitutes the above-mentioned ligand (LI) with a methylamine (¹¹CH₃NH₂) (yielding -CONH¹¹CH₃). Moreover, they can be also introduced by using iodine glucose derivatives (see Japanese Patent Laid-Open No. 09-176179, Japanese Patent Laid-Open No. 09-176050 and Japanese Patent Laid-Open No. 07-267980).

The metal atom which constitutes the complex mentioned above or the metal atom or isotope element contained in the mother nucleus MC, the group AC having affinity with a calcified tissue or the ligand LI is suitably selected according to use.

For use as radioactive diagnostic agents, those which emit gamma ray but do not remarkably injure underlying normal organs or tissues after administration are selected. As radioactive nuclides, those which have gamma ray capable of imaging or those which can be mixed (doped) with a radioactive nuclide that contains gamma ray capable of imaging are preferable. This doping radioactive nuclide may be the same or a different element as long as its chemical properties are sufficiently close to those of beta emitter nuclides and its distribution in the living body according to the present invention is the same as or close to the distribution of the beta emitter.

For use as radioactive therapeutic agents, those which emit beta particles but, after administration, do not remarkably injuring underlying normal organs or tissues while treating affected regions are selected. These radioactive nuclides have an average β energy of 0.25 to 2.75 Mev, but do not need to have gamma ray that enables imaging, and may suitably have an average soft tissue penetration degree of 0.70 to 25.0 mm and a half-life of 0.05 to 700 hours.

Preferable metal atoms and isotope elements include elements selected from the group consisting of the elements of atomic number 6-9, 15-17, 21-29, 31, 35, 37-44, 49, 50, 53, 56-70, 72-75, 81, 83 and 85. Similarly preferable metal atoms and isotope elements include radioactive nuclides selected from the group consisting of 11-C, 15-O, 18-F, 32-P, 59-Fe, 67-Cu, 67-Ga, 81-Rb, 89-Sr, 90-Y, 99m-Tc, 111-In, 123-I, 124-I, 125-I, 131-I, 117m-Sn, 153-Sm, 186-Re, 188-Re, 201-T1, 211-At, 212-Bi and 213-Bi, and among these, preferred are radioactive nuclides selected from the group consisting of 32-P, 59-Fe, 67-Cu, 67-Ga, 81-Rb, 89-Sr, 90-Y, 99m-Tc, 111-In, 123-I, 124-I, 125-I, 131-I, 117m-Sn, 153-Sm, 186-Re, 188-Re, 201-T1 and 212-Bi.

When the compound of the present invention is used for a nuclear magnetic resonance (MRI) diagnostic agent, preferable metal atoms and isotope elements include elements selected from the group consisting of chromium (III), manganese (II), iron (II), iron (III), praseodymium (III), neodymium (III), samarium (III), ytterbium (III), gadolinium (III), terbium(III), dysprosium (III), holmium (III), and erbium (III).

When the compound of the present invention is used for X-ray or ultrasonic diagnosis, preferable metal atoms and isotope elements includes elements selected from the group consisting of bismuth, tungsten, tantalum, hafnium, lantern, lanthanide, barium, molybdenum, niobium, zirconium and strontium.

### (4) Linker

The above-mentioned group AC having affinity with a calcified tissue and the ligand LI may be coupled with the mother nucleus AC by way of a linker L. As such a linker L, can be used polylysine, other peptides, alkyl, polyacrolein, and alkyl ethers, alkylamide, alkylamine and an alkylolefin represented by formula -(CH₂)_{w}-R²⁴-(CH₂)_{w}- (wherein w is each independently 0 to 5, and R²⁴ is O, S, NHCO, NH, or CH=CH), as well as divalent reagents that are used in enzyme immunoassay, etc.

The divalent reagents include, for example, N-succinimidyl-3-(2-pyridyldithio)propionate (SPDP), ethyleneglycol-O,O'-bis(succinimidylsuccinate) (EGS), N-(4-maleimidebutyryloxy)succinimide (GMBS), N-(4-maleimidebutyryloxy)sulfosuccinimide sodium salt (Sulfo-GMBS), N-(6-maleimidecaproyloxy)sulfosuccinimide sodium salt (Sulfo-EMCS), N-(8-maleimidecapryloxy)sulfosuccinimide sodium salt (Sulfo-HMCS), N-(11-maleimideundecanoyloxy)sulfosuccinimide sodium salt (Sulfo-KMUS), 3,3'-dithiobis(sulfosuccinimidylpropionate) (DTSSP), glutaraldehyde, etc.

The reaction of the linker L and the mother nucleus MC, and the reaction of the linker L, the above-mentioned ingredient AC and the ligand LI can be carried out by methods known per se. For example, the reaction for coupling DTPA-mono(2-aminoethylamide) or DTPA-mono(6-aminohexylamide) with an amino group of IgG or Fab fragment through EGS or DTSSP can be performed according to the method of Japanese Patent No. 2815615. In case of using polylysine, the reaction can be performed according to the method of Japanese Patent No. 2548711, and in case of using polyacrolein, the reaction can be performed according to the method of Japanese Patent No. 1729192, and when dialdehyde starch and amino oligosaccharide are used as the mother nucleus MC, the reaction can be performed according to Japanese Patent No. 1721409, Japanese Patent Laid-Open No. 7-206895, etc.

### (5) Preferable compounds

According to preferable embodiment of the present invention, compounds represented by the following general formula V-1 or V-2 are provided. 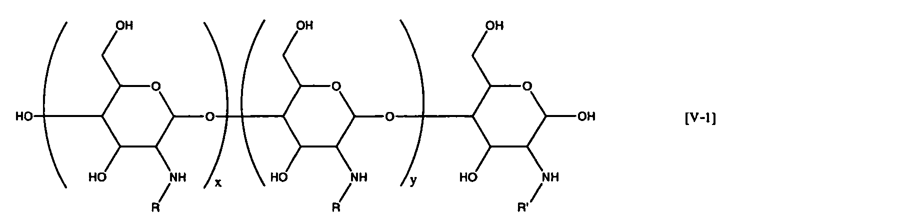 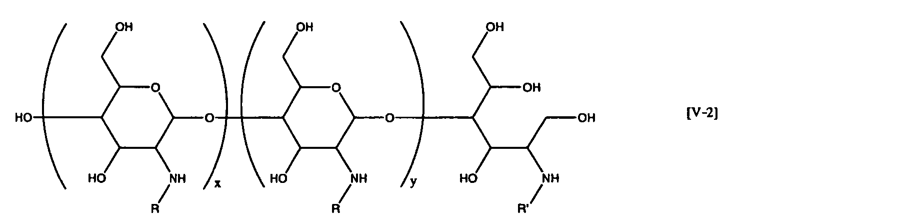 (wherein R and R' are each independently an group AC having affinity with a calcified tissue or a ligand LI for binding to a metal atom, and at least one of them is the group AC having affinity with a calcified tissue; x and y are each independently 0 to 19; and x+y is 1 to 19).

The compound represented by the above-mentioned general formula can be easily obtained by reacting a bisphosphonate compound (BP) as a group AC having affinity with a calcified tissue to an amino group of the aminooligosaccharide consisting of one or more kinds of monosaccharides selected from the group consisting of glucosamine, mannosamine and galactosamine which constitute the mother nucleus MC, and reacting the carboxylic acid group of polyaminopolycarboxylic acid as the ligand LI. In this case, the terminal end of the aminooligosaccharide may be reduced but does not need to be reduced. However, when the terminal end is reduced, the amino group at the terminal end of the aminooligosaccharide can be selectively modified with a protecting group, and consequently a desired compound can be chemically bonded to the amino group at the terminal end of the reduced oligosaccharide and therefore it is convenient. When this reaction is performed, the reduced amino oligosaccharide has preferably 2 to 50 saccharide units, more preferably 2 to 20 saccharide units, and particularly preferably 2 to 13 saccharide units.

In this way, according to another aspect of the present invention, a method of selectively modifying an amino group at a terminal end is provided which comprises providing an amino oligosaccharide having 2 to 50 saccharide units which consists of one or more monosaccharides selected from the group consisting of glucosamine, mannosamine and galactosamine and is reduced at a terminal end thereof, and subjecting the amino oligosaccharide to a reaction for generating a carbamate compound.

In addition, according to still another aspect of the present invention, a method of selectively modifying an amino group at a terminal end with a butoxycarbonyl (Boc) group is provided which comprises reacting, with dibutyl dicarbonate, an aminosaccharide of 2 to 13 saccharide units which consists of one or more monosaccharides selected from the group consisting of glucosamine, mannosamine and galactosamine and is reduced at a terminal end thereof.

The above-mentioned modifying method is convenient for an intermediate of the compound represented by the formula (AC)ₐ-MC which is a compound having affinity with a calcified tissue according to the present invention.

The most preferable compound of the present invention can be represented by the following chemical formulae.

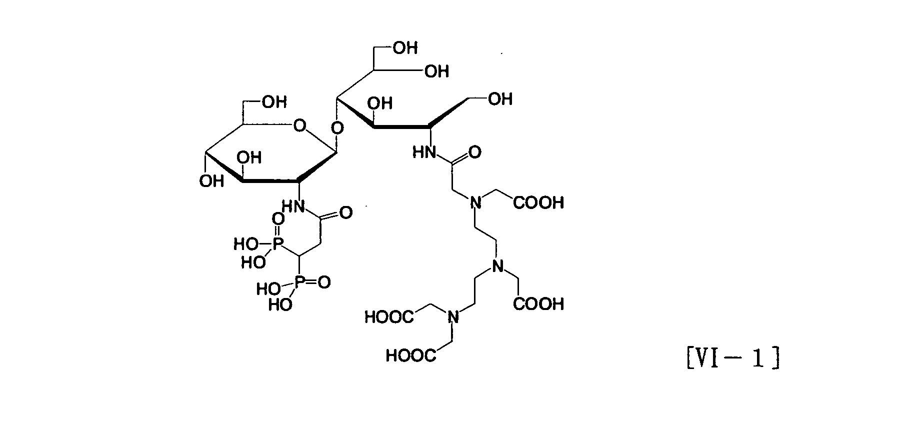

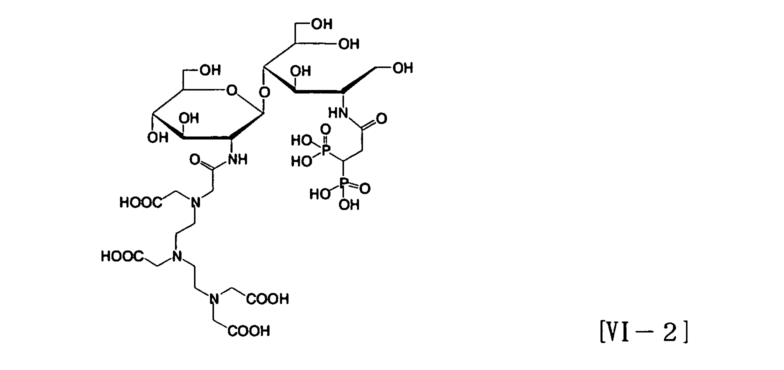

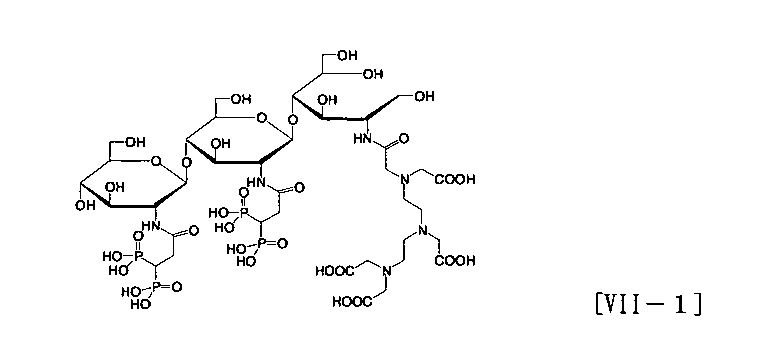

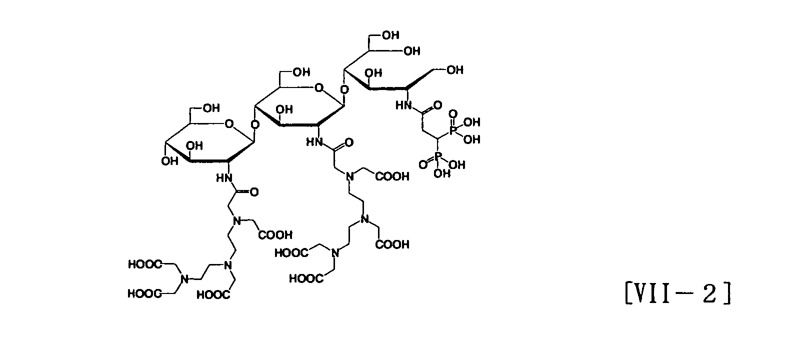

### (6) Formulation, kit and dosage

The compound of the present invention may be in a form of salt, hydrate, and solvate. As the salt, mention may be made of pharmaceutically acceptable salts with inorganic bases such as salts with alkaline metals such as lithium, sodium and potassium, salts with alkaline-earth metals such as salts with calcium and magnesium, ammonium salts, salts with organic bases such as methylamine, ethylamine, dimethylamine, diethylamine, trimethylamine, triethylamine,cyclohexylamine, ethanolamine, diethanolamine, morpholine and meglumine, salts with basic amino acids such as lysine, ornithine and arginine. Preferably sodium and potassium can be suitably used.

The compound of the present invention can be used in a form of an aggregate, an aqueous solution or a lyophilized product. For example, it can be in a form of a kit for preparing a radioactive labeled compound by allowing a lyophilized preparation to co-exist with a reducing agent, a stabilizing agent, etc. The kit for preparing a radioactive labeled compound containing the compound of the present invention is preferably provided as a lyophilized preparation which is dissolved in a suitable dilution agent when it is used, and which is labeled with a radioactive nuclide such as technetium or rhenium metal salt upon administration. Alternatively, it may be in a form of the above-mentioned aqueous solution agent, and labeled with a radioactive transition metal such as technetium or rhenium metal salt by commonly-used drug preparation techniques or in a presence of a non-metallic reducing agent.

As for forms of the compound of the present invention, a form in which the mother nucleus MC or the group AC having affinity with a calcified tissue is substituted with a substituent group which contains a halogen group, a leaving group or a metal alkyl group is convenient. The halogen atom is preferably fluorine, bromine, iodine, etc., and the metal alkyl group includes trialkyl tin represented by the formula Sn(R₃), etc., and the alkyl group includes a methyl group, an ethyl group, a propyl group, a butyl group, etc. Preferably, trimethyltin or tributyltin is be used. The labeling reaction of the substituent group X with a radioactive halogen is performed by known methods, preferably performed by a substitution reaction or exchange reaction.

Conventionally used additives such as an oxidizing agent, stabilizing agent, buffering agent and excipient may be added to the kit for preparing the radioactive compound. For example, chloramine T or hydrogen peroxide as needed is added as an oxidizing agent, followed by the labeling reaction. This radioactive halogen labeling may be performed by a known method, in which temperature, concentration, pH, etc. are not particularly limited.

As a reducing agent used for chemical reduction of peracid ions such as 99m-pertechnetate (99mTcO₄⁻) in performing labeling a radioactive transition metal, can be generally used metal ions such as of tin, zinc and iron, or metallic compounds such as chromium chloride and chromium acetate, including tin chloride, stannous fluoride, etc., but nonmetallic reducing agents such as sodium diphenylphosphinobenzene-3-sulfonate, formamidinesulfonic acid or glucoheptanoic acid can be also used besides the metallic compounds. It is also possible to use dithionic acid and sodium bisulfite. In addition, by using compounds which form a relatively unstable complex and are exemplified by organic acid such as gluconic acid, ascorbic acid and citric acid and a saccharide such as mannose, it is possible to perform a complex exchange reaction with the compound of the present invention and to transfer the radioactive transition metal, thereby performing the labeling. There is no particularly restriction on the reaction conditions such as temperature, concentration, and pH, and usually the reaction can be performed at room temperature or under heating, and a reducing agent is suitably used according to the reaction conditions of these reactions.

When a physiological saline which contains pertechnetate eluted from a 99mTc generator is added to a preparation of the kit according to the present invention that contains a reducing agent, technetium is reduced from heptavalence to the lower number of oxidization by the reducing agent and forms a complex with DTPA. Although the exact character of the formed complex is unknown, it would be possible that the reducing agent can also form a part of these complexes. However, since the compound represented by the formula (AC)ₐ-MC-(LI)_{b} of the present invention has a ligand LI, it is possible to obtain a complex in which the component AC does not participate in complexation due to the difference in coordinating ability between the ligand LI and the component AC. After the liquid is shaken, heated or allowed to stand still in order to ensure completion of reduction and formation of a complex, the liquid can be used for injection.

The compound of the present invention may contain physiologically acceptable buffering agents (for example, pH adjusting agents such as a physiological saline, acetic acid, phosphoric acid, carbonic acid and tris(hydroxymethyl)aminomethane, etc.) and other physiological acceptable additives (for example, stabilizing agents such as ascorbic acid and paraben, dissolution agents such as meglumine and betaine, and excipients such as D-mannitol).

The compound of the present invention can be used in a similar manner to conventional diagnostic agents or therapeutic agents, and, for example, it can be used as a liquid preparation administered to human and other mammals by injection. The dose is substantially the same as that of conventional diagnostic agents or therapeutic agents, and about 3 to 25 MBq/kg, preferably 6 to 12 MBq/kg for diagnostic agents, and depends on radioactive nuclides for therapeutic agents. The dose is suitably varied depending on kinds of compounds, kinds of radioactive nuclides to be used, age, weight, condition of patients, administration methods, and other agents used in combination, and the like.

### Examples

Below, the present invention will be further described in detail by way of Examples, but the present invention is not limited to these Examples. Analysis was performed using methods well-known to those skilled in the art. In the Examples, measurement of NMR spectrum was performed using JNM-500 (manufactured by JEOL) and measurement of MS spectrum was performed using Q-Tof type (manufactured by Micromass). The abbreviations used in Examples are as follows:
WSCD: 1-ethyl-3-(3-diethylaminopropyl)carbodiimide,
HOBt-H₂O: 1-hydroxytriazole monohydrate,
DTPA: diethylenetriaminepentaacetic acid,
HPLC: High Performance Liquid Chromatography,
-OAc: acetyl group,
Boc-: tert-butoxycarbonyl group,
Bn-: benzyl group.

The "desalting" described below was performed under the following conditions:
Electric dialysis equipment: MICROACILYZER G3 (trade name, manufactured by Asahi Chemical Co., Ltd.);
Dialysis membrane: AC-110-400 (trade name, manufactured by Asahi Chemical Co., Ltd.); and
Electrode liquid: 0.35 mol/L aqueous sodium sulfate solution.

### Example 1 (Selective Boc-substitution reaction in chitobiitol and chitotriitol and whole synthesis of VI-1, VI-2, VII-1 and VII-2)

### (1) Synthesis of VI-1

The scheme of synthesis of VI-1 is shown below:

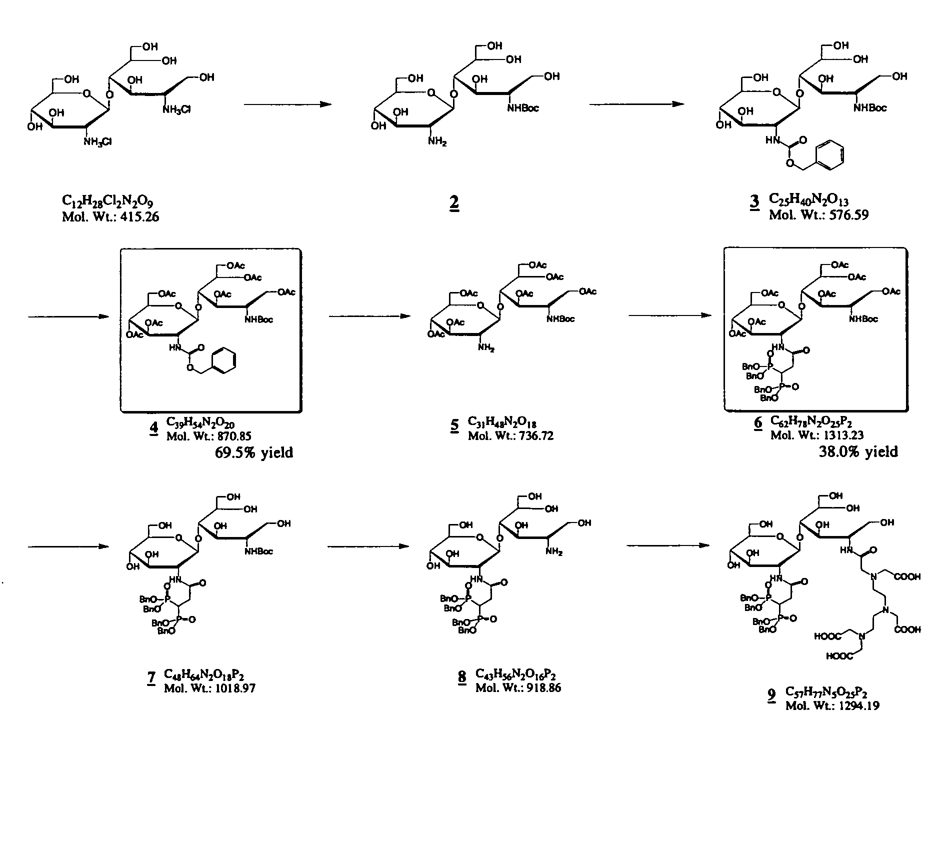

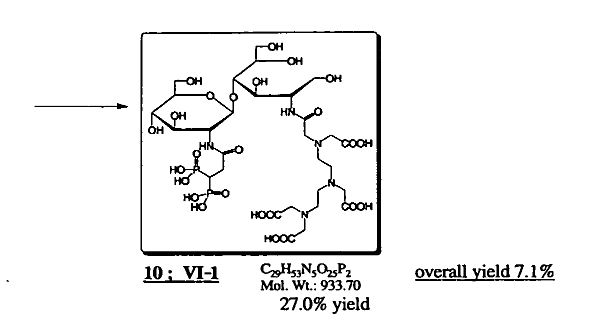

4.16 g (10.0 mmol) of chitobiitol dihydrochloride was measured in a 200-mL eggplant flask, dissolved in 80 mL of water and the resulting mixture was stirred at room temperature. 3.40 g (32.0 mmol) of sodium carbonate was suspended and 60 mL of methanol was added. 2.29 g (10.5 mmol) of dibutyl dicarbonate was added and the resulting mixture was stirred overnight (for about 16.5 hrs). 1.88 g (11.0 mmol) of Z-chloride was added to the reaction solution, and agitation was continued overnight (for about 24 hrs). The solvent of the reaction solution was evaporated to dryness and colorless residue was obtained. The residual substance was dissolved in 60 mL of pyridine, 22 mL of acetic anhydride was added under stir at room temperature, and the resulting mixture was stirred at room temperature overnight (for about 23 hrs). 30 mL of methanol was added to the reaction solution, and the solvent was evaporated under reduced pressure. Oily residue was extracted (150 mL of chloroform × 2/200 mL of water), and after the organic layer was dried over anhydrous sodium sulfate, the solvent was evaporated to obtain 9.37 g of residual substance. The residual substance was subjected to silica gel chromatography (Silica gel 60 250 g, hexane/ethyl acetate/methanol =6/4/1), and 6.06 g (6.95 mmol, yield: 69.5%) of "Compound 4" was obtained.

4.20 g (4.82 mmol) of Compound 4 was measured in a 100 mL eggplant flask, and 421 mg (10 w/w%) of palladium-carbon (act.10%) and 80 mL of methanol were added. The mixture was subjected to catalytic reduction under hydrogen gas atmosphere under agitation at room temperature. After the reaction ended, filtration with glass filter was carried out, and after washed with methanol, the solvent of the filtrate was evaporated and 3.41 g of residual substance was obtained. To this compound, 3.45 g (5.80 mmol) of 3,3- bis(dibenzyloxyphosphoryl)propanoic acid obtained by a known method, for example, the method of Page PCB et al. (Page PCB, et al., J. Org. Chem, 66, 3704-3708, 2001) was added, dissolved in 50 mL of methylene chloride, and 929 mg (5.80 mmol) of WSCD and 789 mg (5.84 mmol) of HOBt·H₂O were added under agitation in an ice bath, and agitation was continued overnight (for about 19.5 hrs). The solvent of the reaction liquid was evaporated, the residual substance was subjected to silica gel chromatography (Silica gel 60 250 g, hexane/ethyl acetate/methanol =6/4/1), and 2.41 g (1.83 mmol, yield: 38.0%) of "Compound 6" was obtained.

The following numbers were assigned to carbon atoms in the analysis by ¹H-nmr and ¹³C-nmr of "Compound 6." 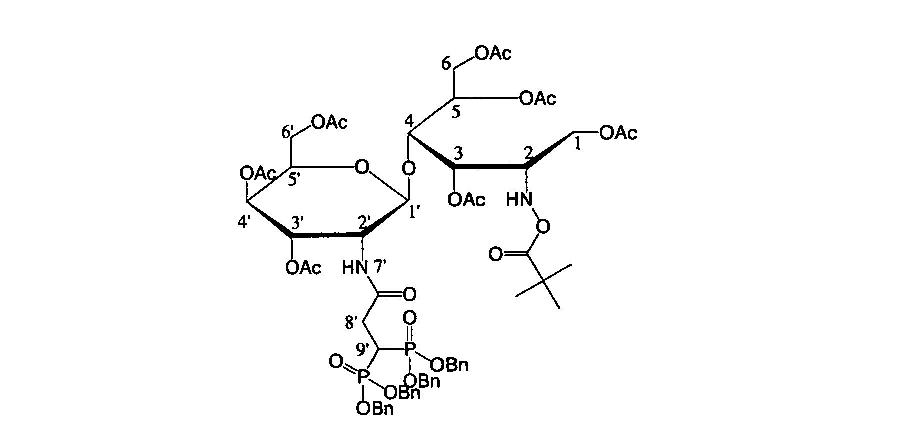 ¹H-nmr(500MHz, CDCl₃, δ) : 1. 4 (9H, s, -C (CH₃)₃), 1.82 (3H, s, -OAc), 1.94(3H, s, -OAc), 1.98(3H, s, -OAc), 1.99(3H, s, -OAc), 2.02(3H, s, -OAc), 2.10(3H, s, -OAc), 2.64~2.84 (1H, 9'), 3. 30~3.44 (2H, 8'), 3.62~3.70 (1H, 5'), 3.88~3.96 (1H, 1), 3.88~3.96 (1H, 4), 5.22~ 5.36(1H, 5), 3. 88~3.96 (1H, 2'), 4. 06~4.20 (1H, 1), 4. 06~4.20 (1H, 6), 4. 06~4.20 (2H, 6'), 4.26~4.38 (1H, 2), 4. 46~4.54 (1H, 6), 4.88~ 5.20(1H, 3), 4.88~5.20(1H, 4'), 4.88~5.20(8H, m, -OCH₂-φ), 5.10~ 5.16(1H, 1'), 5.22~5.36(1H, 3'), 7.3(20H, m, -φ).
¹³C-nmr (500MHz, CDCl₃, δ) : 20. 45 (-COCH₃), 20.54(-COCH₃), 20.65(-COCH₃), 20.70(-COCH₃), 28.27(-COCH₃), 31. 68 (br s, 8'), 32. 13 (t, J=Hz, 9') , 48.90, 55.20, 62.32, 62.40, 63.11, 68.10, 68.15, 68.24, 68.29, 68.35, 68.40, 68.64, 69.35, 69.43, 69.53, 72.14, 72.28, 74. 25, 80.30, 98.97(1'), 127.97(-Aromatic), 128. 11 (-Aromatic), 128. 26 (-Aromatic), 128. 44 (-Aromatic), 135. 95 (d, J=Hz, -Aromatic), 155. 77, 169. 33, 169.59, 169.70, 169.76, 170.27, 170.45, 170.54, 170.71, 170. 78.

600 mg (0.46 mmol) of Compound 6 was measured in a 50-mL eggplant flask, dissolved in 20 mL of methanol, and 126 mg (2.33 mmol) of sodium methoxide was added under agitation at room temperature. After the reaction ended, the reaction liquid was passed through 4.57 g of AG™-50W-X8 resin (trade name, manufactured by Nippon Bio-Rad Laboratories, Inc.), washed out with methanol and water, and the solvent was evaporated to obtain 460 mg (0.451 mmol as Compound 7) of residual substance. The residual substance (Compound 7) was dissolved in 7 mL of methanol, and 4.0 mL of 10% hydrochloric acid-methanol was added under agitation at room temperature. After 3 hours, the solvent was evaporated and 392 mg (0.427 mmol as Compound 8) of residual substance was obtained. To 392 mg (0.427 mmol as Compound 8) of residue, DTPA was introduced by a known method, for example, the method of Takahashi et al. [Japanese Patent Laid-Open 2002-187948]. It was dissolved in 7.6 mL of water and 2.41 g of 8 N sodium hydroxide, and warmed to 80°C. While keeping the temperature constant, 1.61 g (4.51 mmol) of anhydrous DTPA was added to the solution for 3 minutes and warming under agitation was continued for 30 minutes. After cooled to 30°C, 0.71 g of 8 N sodium hydroxide was added to adjust pH to 9.0, and it was warmed to 80°C again, and warming under agitation was carried out for 30 minutes. Then, the mixture was cooled to 30°C, and 0.5 mL of 6 N hydrochloric acid was added to adjust pH to 8.0 to terminate the reaction. The solvent of the reaction liquid was evaporated, 9.5 mL of water was added to dissolve it and 831 mg (200 w/w%) of palladium-carbon (act.10%) was added and the mixture was subjected to catalytic reduction under hydrogen gas atmosphere for 1.5 hours under agitation at room temperature. After the reaction ended, filtration with glass filter was carried out, the solvent of the filtrate was evaporated, and the residue was obtained. The residue was separated and purified by recycling HPLC, and after desalination treatment, the solvent was evaporated and 115 mg (0.123 mmol; yield: 27.0%) of "Compound 10; VI-1" was obtained.

The following numbers were assigned to carbon atoms in the analysis by ¹H-nmr and ¹³C-nmr of "Compound VI-1." 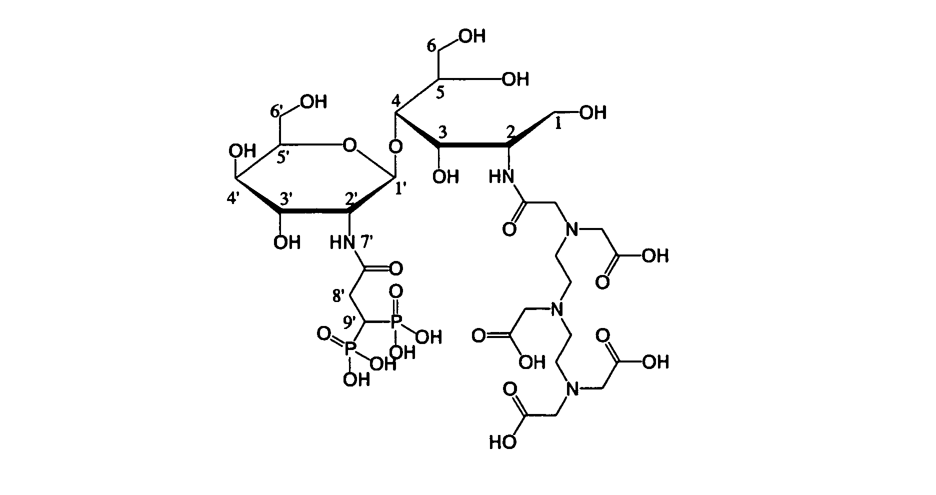 ¹H-nmr (500MHz, D₂O, TSP) : 2. 30 ~ 2. 50 (9'), 2. 60 ~ 2. 80 (8') , 3. 1 ~ 3. 4 (DTPA), 3. 39 ~ 3. 46 (5'), 3. 46 ~ 3. 52 (4') , 3. 52 ~ 3. 60 (5), 3. 60 ~ 3. 69 (1), 3. 61 ~ 3. 69 (3'), 3. 70 ~ 3. 75 (2') , 3. 70 ~ 3. 75 (6) , 3. 70 ~ 3. 83 (1), 3. 75 ~ 3. 82 (4), 3. 75 ~ 3. 82 (6') , 3. 83 ~ 3. 87 (6) , 3. 87 ~ 3. 92 (3), 3. 87~3. 93 (6'), 4. 35~4. 40 (2) , 4. 62~4. 68 (1'), 8. 4 (7 or 7'), 8. 7 (7' or 7).
¹³C-nmr (500MHz, D₂O, TSP) : 33. 49 (8') , 36. 41 (t, J=136Hz; 9') , 51. 16, 51. 74, 52. 00, 53. 12, 55. 85, 56.67, 57. 90, 58. 16, 58. 43, 60.74, 61. 22, 62. 29, 68. 13, 69. 30, 71. 30, 73. 85, 75. 86, 79. 01, 101. 14 (1'), 172. 02, 173.92, 174. 31, 175. 93, 175.98, 176. 02, 176. 07, 176.85. MS (ESI-) : 932. 2427 C₂₉H₅₂N₅O₂₅P₂ requires 932. 2477 ([M-H]⁻).

### (2) Synthesis of VI-2

The scheme of synthesis of VI-2 is shown below:

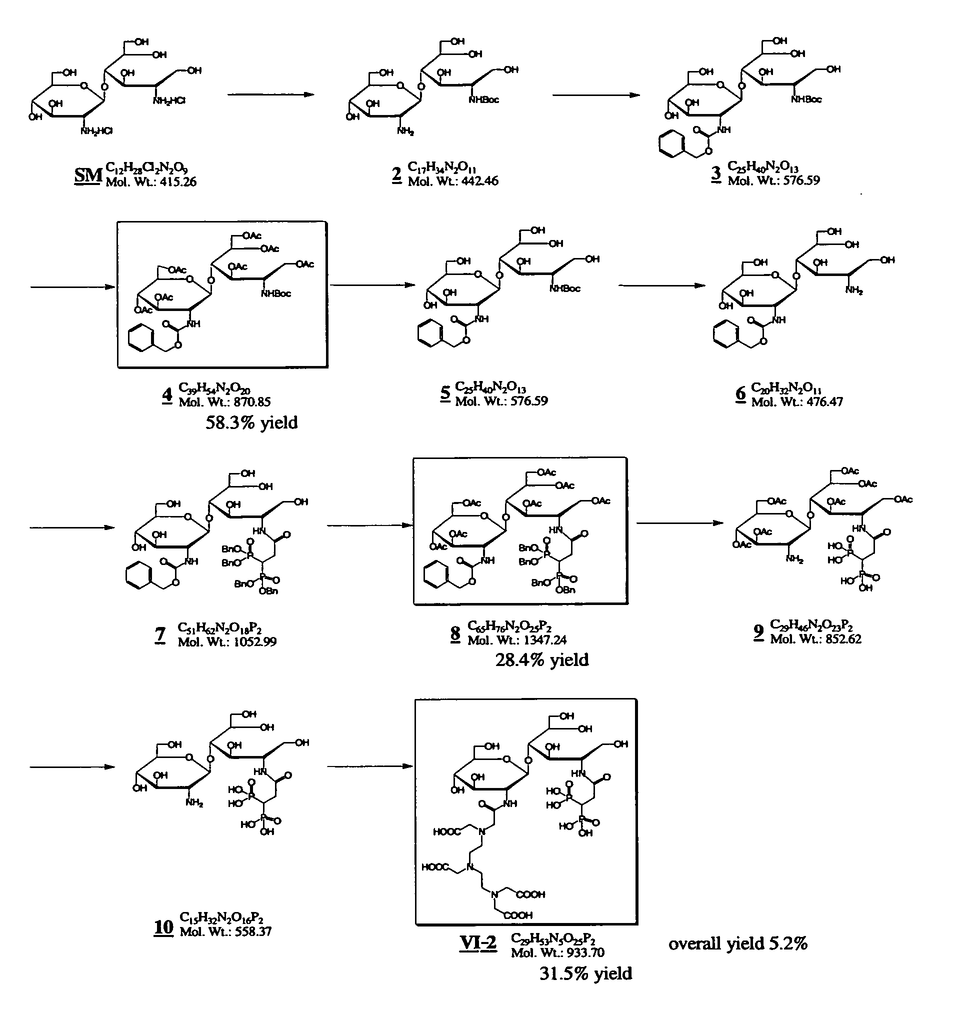

15.0 g (36.1 mmol) of chitobiitol dihydrochloride was measured and dissolved in 280 mL of water, and 12.3 g (116 mmol) of sodium carbonate was suspended, and 210 mL of methanol was added, and the mixture was stirred at room temperature. 8.71 mL (38.0 mmol) of dibutyl dicarbonate was added and the resulting mixture was stirred overnight at room temperature. 5.7 mL (40.0 mmol) of Z-chloride was added, and agitation was conducted overnight at room temperature. The solvent of the reaction liquid was evaporated, 217 mL of pyridine was added to the residual substance, and 79.4 mL (842 mmol) of acetic anhydride and a catalytic amount of 4-(N,N-dimethylamino) pyridine were added in an ice bath, and the resulting mixture was stirred overnight. 108 mL of methanol was added to the reaction solution in an ice bath, and the solvent was evaporated. The residue was extracted (chloroform/saturated potassium bisulfate aqueous solution, saturated sodium bicarbonate aqueous solution), and the organic layer was washed with water and dried over anhydrous sodium sulfate, and filtrated after the solvent was then evaporated. The obtained crude product was subjected to column chromatography (Silica gel N60 (trade name, manufactured by Kanto Chemical Co., Inc.), ethyl acetate/hexane/methanol = 4/6/1), and the solvent was evaporated to obtain 18.3 g (21.0 mmol, yield: 58.3%) of "Compound 4."

4.35 g (5.0 mmol) of Compound 4 was measured, and dissolved in 50 mL of methanol, and 1.35 g (25.0 mmol) of sodium methoxide was added, and agitation at room temperature was carried out for 2 hours. 50.0 g of washed AG™-50W-X8 resin (trade name, manufactured by Nippon Bio-Rad Laboratories, Inc.) was added. It was succeedingly stirred for 30 minutes and filtered, and the solvent of the filtrate was evaporated. It was redissolved in 21 mL of methanol, and 12 mL of 10% hydrochloric acid-methanol solution (manufactured by Tokyo Kasei Kogyo Co., Ltd.) was added. The solvent was evaporated and the residual substance was obtained after carrying out agitation at room temperature for 3 hours. 3.57 g (6.00 mmol) of 3,3- bis(dibenzyloxyphosphoryl)propanoic acid was added to this residual substance, and 50 mL of dimethylformamide was added and was stirred. 1.10 mL (6.26 mmol) of WSCD and 811 mg (6.00 mmol) of HOBt-H₂O were added one by one under argon atmosphere in an ice bath, and the mixture was stirred overnight. The solvent was evaporated, 30 mL of pyridine was added, a catalytic amount of 4-(N,N-dimethylamino)pyridine was added, and 12.0 mL (127 mmol) of acetic anhydride was added. After it was stirred overnight, 15 mL of methanol was added and extraction (chloroform/saturated potassium bisulfate aqueous solution, saturated sodium bicarbonate aqueous solution) was performed. After the organic layer was washed with water and dried over anhydrous sodium sulfate, the solvent was evaporated to obtain the residual substance (about 7.7 g). This residual substance was subjected to column chromatography (Silica gel N60 (trade name, manufactured by Kanto Chemical Co., Inc.) about 200 g, hexane/ethyl acetate/methanol =4/6/1), and the crude product fractions 1, 2, and 3 were obtained. Fractions 1 and 2 were subjected to preparative TLC (Cat. No. 105717 manufactured by Merck Co., hexane/ethyl acetate/methanol =5/5/1), and 741.6 mg (490.9 mg from Fraction 1, 250.7 mg from Fraction 2) of "Compound 8" was obtained. Fraction 3 was again subjected to column chromatography (Silica gel N60 (manufactured by Kanto Chemical Co., Inc.) about 100 g, hexane/ethyl acetate/methanol =10/5/1), and 1.17 g (0.498 mmol, yield: 14.6%) of "Compound 8" was obtained. 1.91 g (1.42 mmol, yield: 28.4%) of "Compound 8" was obtained from this reaction in total.

1.10 g (0.816 mmol) of Compound 8 was dissolved in 10 mL of methanol, and 309 mg (5.71 mmol) of sodium methoxide was added under argon atmosphere, and was stirred at room temperature for 2 hours. 9.0 g of AG™-50W-X8 resin (trade name, manufactured by Nippon Bio-Rad Laboratories, Inc.) was added and agitation was continued for 30 minutes. The reaction mixture was filtered, the solvent of the filtrate was evaporated and 551 mg of residual substance was obtained. 15 mL of methanol and 5 mL of saturated sodium bicarbonate aqueous solution were added to the residual substance. 400 mg of palladium-carbon (act.10%) was added, and the mixture was stirred under hydrogen atmosphere, and subjected to catalytic reduction for 2 hours. After the reaction ended, it was filtered and the filtrate was evaporated. 5 mL of water and 4.17 mL of 8 mol/L sodium hydroxide aqueous solution were added to this residual substance and the mixture was stirred. It was heated to 80°C, 2.97 g (8.20 mmol) of anhydrous DTPA was added, and agitation was continued for 30 minutes. After allowed to room temperature, 8 mol/L sodium hydroxide aqueous solution was added to adjust to pH 9, and the mixture was warmed to 80°C again for 30 minutes. It was allowed to room temperature, and adjusted to pH 8 and concentrated. This concentrated liquid was separated and purified by recycling HPLC (column: Shodex Asahipak GS 320-21G (trade name, manufactured by Showa Denko K.K., 21.5 mm ID × 500 mm) and Shodex Asahipak GS 220-21G (trade name, manufactured by Showa Denko K.K., 21.5 mm ID × 500 mm) serially connected, mobile phase: 100 mmol/L sodium chloride aqueous solution, flow rate: 5.0 mL/min, detector: visible-ultraviolet absorptiometer (detection wavelength: 210 nm)). Desalting was performed on the separated solution, and the solvent was evaporated to isolate 239.9 mg (0.257 mmol, yield: 31.5%) of "Compound VI-2."

The following numbers were assigned to carbon atoms in the analysis by ¹H-nmr and ¹³C-nmr of "Compound VI-2." 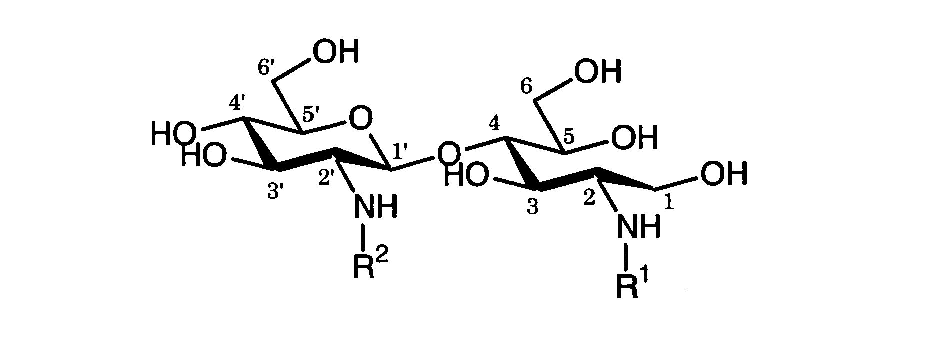 ¹H-nmr (500Hz, D₂O, TSP) : 3.80(1H, m, 1) , 3.65(1H, m, 1), 4.30(1H, m, 2), 3.89(1H, m, 3), 3.82(1H, m, 4), 3.83(1H, m, 5), 3.71(1H, m, 6), 3.53(1H, m, 6), 4.66(1H, d, J=8.2Hz, 1'), 3.79(1H, m. 2'), 3.71(1H, m, 3'), 3.51(1H, m, 4'), 3.48(1H, m, 5') , 3.92(1H, m, 6'), 3.81(1H, m, 6'), 2.72 (dd, ³J_{H-P}=15.1Hz, J=6.9Hz, -COCH₂CH-), 2. 39 (dd, ²J_{H-P}=21.1Hz, J=6.9Hz, -COCH₂CH-), 3.65(2H, m), 3.62(2H, s), 3.39(2H, s), 3.62(2H, s), 3.29(2H, s) , 3.22(2H, m) , 3.08(2H, m).
¹³C-nmr (500MHz, D₂O, TSP) : 61.30(1), 53.67(2), 68.57(3), 78. 47 (4) , 71.42(5), 62.33(6), 101.28(1' ), 56.00(2' ), 73. 56 (3' ) , 69.98(4' ), 75.86(5'), 60.77(6' ), 176.01(t, ³J_{C-P}=8.6Hz), 33.70, 36.78(t, ²J_{C-P}=155.2Hz), 173.66, 177.86, 174.12, 174.66, 58.65, 58.73, 56.00, 58.10, 52.35, 51.51, 51. 12.
³¹P-nmr (500MHz, D₂O) : 19.43, 19. 38.
MS (ESI-) : 1066. 1454 C₂₉H₄₈N₅O₂₅P₂Na₆ requires 1466. 1500[[M·Na₅+Na]⁻.

### (3) Synthesis of VII-1

The scheme of synthesis of VII-1 is shown below:

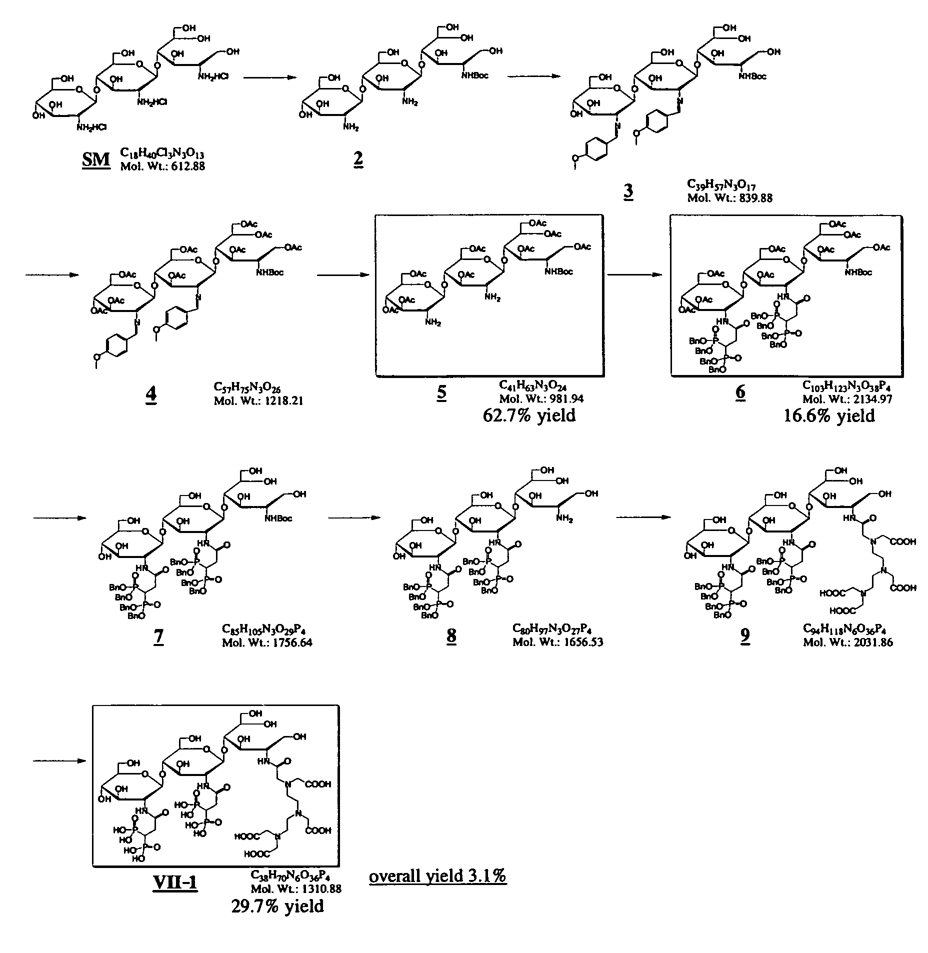

3.08 g (5.02 mmol) of chitotriitol trichloride was measured in a 200 mL eggplant flask, 2.24 g (21.10 mmol) of sodium carbonate was added, and 50 mL of methanol and 36 mL of water were added and the mixture was stirred. 1.4 mL (6.09 mmol) of dibutyl dicarbonate was added, then, after stirring for 14 hours the solvent of the reaction mixture solution was evaporated. 58 mL of methanol was added to the residual substance and was stirred, and 1.45 mL (11.92 mmol) of p-methoxybenzaldehyde was added, and the mixture was stirred for 24 hours. The solvent of the reaction mixture solution was evaporated, 43 mL of pyridine was added to the residual substance, and it was stirred in a water bath, and 11.3 mL (11.98 mmol) of acetic anhydride was added, and it was stirred for 17 hours. 21 mL of methanol was added on an ice bath and agitation was continued for 15 more minutes. The solvent of the reaction mixture solution was evaporated, and extracted (150 mL of chloroform /100 mL of water ×2) organic layer was dried over anhydrous sodium sulfate, and the solvent was evaporated. 20 mL of dimethylformamide was added to the residue, and after the residue was dissolved, the solvent was evaporated and the residual substance (about 7.2 g) was obtained. 25 mL of ethyl acetate was added to the residual substance, 25 mL of 1 mol/L hydrochloric acid was added under agitation at room temperature, and agitation was continued for 1 hour. The reaction liquid was washed with ethyl acetate (25 mL×4), a basic aqueous solution (15 mL of 5% sodium acetate aqueous solution + 35 mL of saturated sodium carbonate aqueous solution) was added, and adjusted to pH 8 and extracted (chloroform 50 mL×2). The organic layer was washed with 100 mL of saturated sodium bicarbonate, dried over anhydrous sodium sulfate, and the solvent was evaporated to obtain 3.09 g (3.14 mmol, yield: 62.7%, pale yellow crystal) of "Compound 5."

5.62 g (9.45 mmol) of 3,3- bis(dibenzylphosphoryl)propanoic acid and 853 mg (6.31 mmol) of HOBt·H₂O were added to 3.09 g (3.14 mmol) of "Compound 5," and dissolved in 70 mL of dimethylformamide. 1.65 mL (9.39 mmol) of WSCD was added in an ice bath under agitation, and the resulting mixture was stirred for 17 hours. The solvent of the reaction mixture solution was evaporated and extracted (chloroform 150 mL/saturated sodium bicarbonate aqueous solution 100 mL, water 100 mL + saturated sodium chloride aqueous solution 20 mL×3, saturated sodium chloride aqueous solution 100 mL), and the organic layer was dried over anhydrous sodium sulfate and the solvent was evaporated and the residual substance (about 7.4 g, pale yellow crystal) was obtained. The residual substance was subjected to column chromatography (Silica gel N60 (trade name, manufactured by Kanto Chemical Co., Inc.) 250 g, chloroform/methanol = 20/1), and the crude product (Fr 2-3; 1.30 (yellow oily substance) and Fr 4-6; 842 mg (pale yellow crystal)) were obtained from Fractions 2 to 3 (about 600 to 900 mL; 300 mL) and Fractions 4 to 6 (about 900 to 1200 mL; 300 mL). After the crude product of Fraction 2 to 3 was subjected to column chromatography (Silica gel N60 (trade name, manufactured by Kanto Chemical Co., Inc.) 100 g, hexane/ethyl acetate/methanol =12/8/1) to elute high polarity impurities by 1000 mL of mobile phase and then the object fraction was eluted by chloroform/methanol = 10/1, and 831 mg (pale yellow crystal) of "Compound 6" was obtained. In the meantime, the crude product of Fractions 4 to 6 was subjected to column chromatography (Silica gel N60 (trade name, Manufactured by Kanto Chemical Co., Inc.) 100 g, ethyl acetate/chloroform/methanol =10/5/1) and 286 mg (pale yellow crystal) of "Compound 6" was obtained from fractions 11 to 13 (ca. 280 to 340 mL). 1.12 g (0.523 mmol, yield: 16.6%) of "Compound 6" was obtained from this reaction in total.

The following numbers were assigned to carbon atoms in the analysis by ¹H-nmr of "Compound 6." 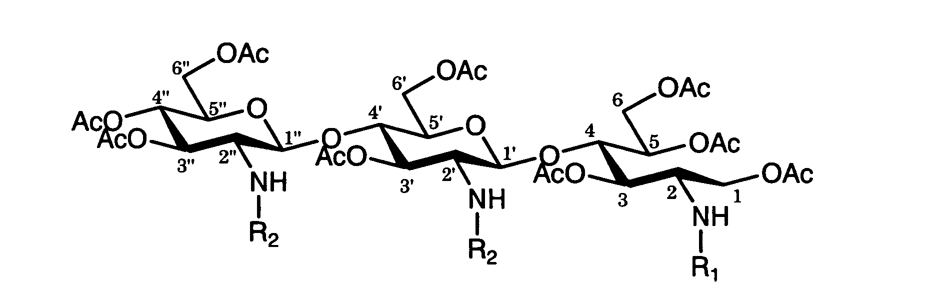 ¹H-nmr (500MHz, CDCl₃, δ) : 4.42(1H, m, 1), 3.92(1H, m, 1), 4.32(1H, m, 2), 5.05(1H, m, 3), 3.94(1H, m, 4), 5.29(1H, m, 5), 4.49(1H, dd, J=12, 2Hz, 6), 4. 12(1H, dd, J=12, 6Hz, 6), 4.92(1H, d, J=10Hz, 2-NH) , 4.81(1H, br. d, H=7Hz, 1'), 3.94(1H, m, 2'), 5.09(1H, t, J=10Hz, 3'), 3.63(1H, t, J=10Hz, 4'), 3.46(1H, m, 5'), 4.31(1H, m, 6'), 4.25(1H, m, 6'), 4.53(1H, d, J=8Hz, 1"), 3.88(1H, m, 2") , 5.16(1H, t, J=10Hz, 3"), 5. 02 (1H, m, 4"), 3. 60 (1H, m, 5"), 4. 36 (1H, dd, J=13, 4Hz, 6"), 3. 99 (1H, dd, J=13, 2Hz, 6") , 6. 57 (1H, d, J=9Hz, 2"-NH) , 2. 08 (3H, s, 6'-Ac), 2. 05 (3H, s, 6"-Ac), 2. 03 (3H, s, 1-Ac), 1. 982 (3H, s, 4"-Ac), 1. 977 (3H, s, 6-Ac), 1. 96 (3H, s, 3-Ac), 1. 95 (3H, s, 5'-Ac), 1. 85 (3H, s, 3'-Ac), 1. 81 (3H, s, 3"-Ac), 1. 41 (9H, s, - C (CH₃)₃), 2. 68~2. 89 ((2H, m, -COCH₂CH-), 3. 28~3. 46 (1H, m, -COCH₂CH-), 7. 24~7. 32 (20H, -CH₂-Ar), 4. 92~5. 05 (8H, -CH₂-Ar)
¹³C-nmr (500MHz, CDCl₃, δ) : 63. 18, 49. 02, 69. 44, 74. 01. 69. 29, 62. 30, 99. 14, 54. 86, 72. 52, 75. 95, 72.80, 62. 10, 100. 95, 54. 86, 72. 25, 67. 89, 71. 79, 61. 51, 171. 31 (6'-Ac), 170. 90 (3"-Ac) , 170. 66 (3'-Ac) , 170. 49 (1-Ac, 6"-Ac), 170. 46 (3-Ac, 5-Ac), 169. 53 (6-Ac), 169. 24 (4"-Ac), 20. 38 ~ 20. 68 (-CH₃), 155. 51, 80. 01, 28. 23, 169. 44 ~ 169. 64, 31. 69 (m), 31. 49 (m), 32. 08 (t, J_{CP}=135Hz), 31. 98 (t, J_{CP}=134Hz), 135. 80~ 136. 05 (-CH₂-Ar), 127. 80~128. 05 (-CH₂-Ar), 68. 05~68. 35 (-CH₂-Ar)
³¹P-nmr (500MHz, CDCl₃) : 24. 54 (d, J_{PP}=3Hz), 24. 19 (d, J_{PP}=4Hz), 24. 15 (d, J_{PP}=3Hz), 24. 11 (d, J_{PP}=4Hz).

650 mg (3.28 mmol) of Compound 6 was dissolved in 20 mL of methanol in a 100 mL eggplant flask, and 138 mg (2.65 mmol) of sodium methoxide was added under argon atmosphere, and the mixture was stirred at room temperature for 1 hour. 4.0 g of AG™-50W-X8 resin (trade name, manufactured by Nippon Bio-Rad Laboratories, Inc.) was added and 20 mL of water was added, and agitation was continued for 15 minutes. The reaction liquid was filtered, the filtrate was evaporated and 551 mg of residual substance was obtained. The residual substance was dissolved in 10 mL of methanol, 5 mL of 10% hydrochloric acid-methanol (manufactured by Tokyo Kasei Kogyo Co., Ltd.) was added, and agitation at room temperature was carried out for 3 hours. The solvent of the reaction liquid was evaporated and 481 mg of residual substance was obtained. 4.43 mL of water and 1.63 mL of 8 mol/L sodium hydroxide aqueous solution were added to this residual substance and the mixture was stirred. It was heated to 80°C, 1.11 g (3.11 mmol) of anhydrous DTPA was added, and agitation was continued for 30 minutes. It was allowed to room temperature, and 8 mol/L of sodium hydroxide aqueous solution was added, adjusted to pH 9, and warmed to 80°C again for 30 minutes. It was allowed to room temperature and adjusted to pH 8. The reaction liquid was washed with chloroform to remove insoluble matters, and a catalytic amount of palladium-carbon (act.10%) was added to the aqueous phase, and the resulting mixture was stirred under hydrogen atmosphere and subjected to catalytic reduction for 3 hours. After the reaction ended, filtration was performed and the filtrate was condensed. This condensed liquid was separated and purified by recycling HPLC (column: Shodex Asahipak GS 320-21G (trade name, manufactured by Showa Denko K.K., 21.5 mm ID × 500 mm) and Shodex Asahipak GS 220-21G (trade name, manufactured by Showa Denko K.K., 21.5 mm ID × 500 mm) serially connected, mobile phase: 100 mmol/L sodium chloride aqueous solution, flow rate: 5.0 mL/min, detector: visible-ultraviolet absorptiometer (detection wavelength: 210 nm)). Desalting was performed on the separated solution, and the solvent was evaporated to isolate 119 mg (0.09 mmol, yield: 29.7%) of "Compound VII-1."

The following numbers were assigned to carbon atoms in the analysis by ¹H-nmr of "Compound VII-1." 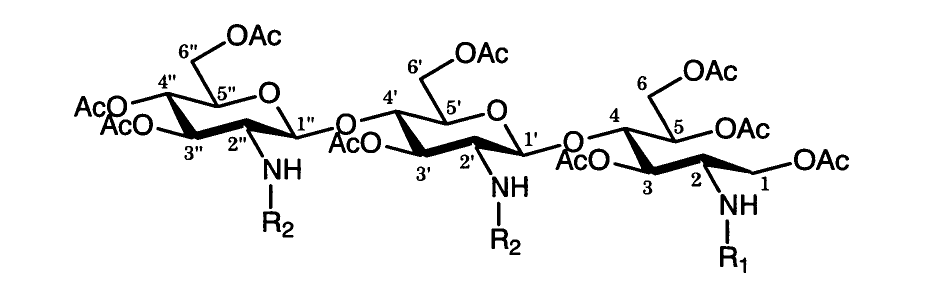 ¹H-nmr(500Hz, D₂O, TSP) : 3.81(1H, m, 1), 3.68(1H, m, 1), 4.38(1H, m, 2) , 3.91(1H, m, 3) , 3.70(1H, m, 4), 3.88(1H, m, 5), 3.79(1H, m, 6), 3.57(1H, m, 6), 4.64(1H, d, J=8Hz, 1'), 3.82(1H, m, 2') , 3.76(1H, m, 3'), 3.71(1H, m, 4'), 3.54(1H, m, 5'), 3.91(1H, m, 6'), 3.72(1H, m, 6'), 4.69(1H, d, J=8Hz, 1"), 3.73(1H, m, 2"), 3.67(1H, m, 3"), 3.47(1H, m, 4"), 3.51(1H, m, 5"), 3.92(1H, m, 6"), 3.75(1H, m, 6"), 4.00(2H, s, -NHCOCH₂-), 3.75(2H, s, -NCH₂CO₂H), 3.62(2H, s, - NCH₂CO₂H), 3. 89 (4H, s, -NCH₂CO₂H×2). 3.39(2H, m, -NCH₂CH₂N-), 3.23(2H, m, -NCH₂CH₂N-), 3.32(2H, m, -NCH₂CH₂N-), 3.56(2H, m, - NCH₂CH₂N-), 2.77(2H, m, -COCH₂CH-), 2.57(1H, m, -CH₂CH-).
¹³C-nmr(500Hz, D₂O, TSP) : 61.09, 53.38, 68.22, 78.82, 71.30, 62.29, 101.07, 56.04, 72.53, 78.33, 74.66, 60.29, 100.95, 56.65, 73.91, 69.58, 76.20, 60.84, 168.60, 172.80, 174.30, 170.65, 57.49, 55.26, 57.63, 52.53, 51.06, 50.00, 52.25, 175.19~175.31 (m), 33.28, 33.12, 36.18(t, J_{CP}=189Hz), 35.67(t, J_{CP}=120Hz).
³¹P-nmr (500Hz, D₂O) : 19.60, 19.39, 19.31, 18. 85.

### (4) Synthesis of VII-2

The scheme of synthesis of VII-2 is shown below:

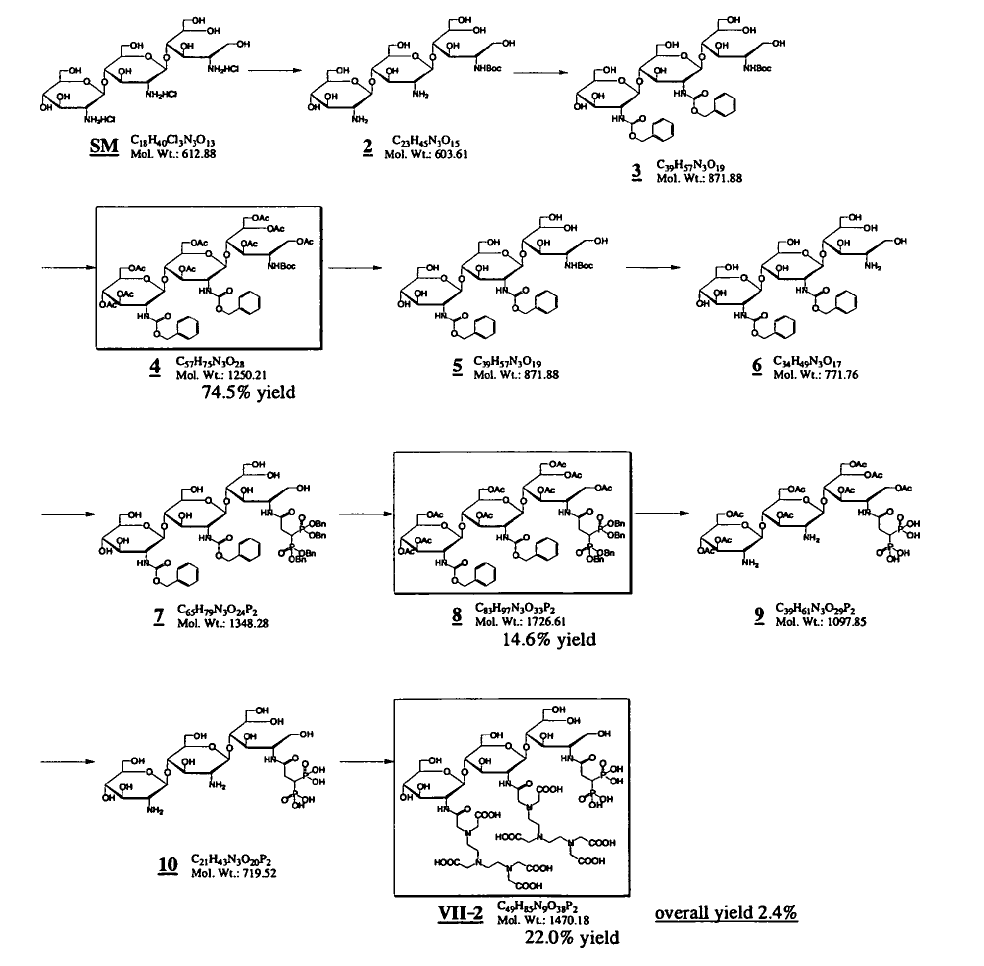

10.0 g (16.3 mmol) of chitotriitol trichloride salt was measured in a 300 mL eggplant flask, dissolved in 160 mL of water and stir at room temperature was carried out. 16.4 g (155 mmol) of sodium carbonate was suspended, and 80 mL of methanol was added. 5.34 g (24.5 mmol) of dibutyl dicarbonate was added, and the mixture was stirred at room temperature for 41 hours. 13.1 g (81.6 mmol) of Z-chloride was added, and the mixture was stirred at room temperature further for about 24.5 hours. After the solvent of the reaction liquid was evaporated, the residual substance was suspended in 100 mL of pyridine. 50 mg of 4-(N,N-dimethylamino)pyridine which is a catalytic amount and 40.0 g (392 mmol) of acetic anhydride were added to this in an ice bath, the reaction liquid was allowed to room temperature, and the mixture was stirred for 21 hours. After 10 mL of methanol was added to the reaction solution in an ice bath and agitated for 10 more minutes, the mixture was poured into 300 mL of saturated sodium bicarbonate aqueous solution by small portions, and extracted (chloroform 200 mL×3). After the organic layer was washed with 50 mL of a saturated sodium chloride solution and dried over anhydrous sodium sulfate, it was filtered and the solvent was evaporated. 23.4 g of the obtained crude product was subjected to column chromatography (Silica gel N60 (trade name, manufactured by Kanto Chemical Co., Inc.) 250 g, ethyl acetate/hexane/methanol = 8/12/1 → 8/12/2), and 15.2 g (12.2 mmol, yield: 74.5%) of "Compound 4" was obtained.

4.26 g (3.41 mmol) of Compound 4 was measured in a 100 mL eggplant flask, and dissolved in 50 mL of methanol, 56.6 mg (1.05 mmol) of sodium methoxide was added, and agitation at room temperature was carried out for 2 hours. After 5.0 g of washed AG™-50W-X8 resin (trade name, manufactured by Nippon Bio-Rad Laboratories, Inc.) was added and the resulting mixture was stirred for 20 minutes succeedingly, filter filtration was carried out, and the solvent of the filtrate was evaporated. 80 mL of methanol was redissolved under argon atmosphere, and 20.0 mL of 10% hydrochloric acid-methanol (manufactured by Tokyo Kasei Kogyo Co., Ltd.) was added, and after agitation was carried out at room temperature for 1 hour, the solvent was evaporated and 3.09 g of residual substance was obtained. 2.56 g (4.31 mmol) of 3,3- bis(dibenzyloxyphosphoryl)propanoic acid was measured in a 100 mL eggplant flask, and 3.09 g of the above residual substance dissolved in 30 mL of dimethylformamide was added thereto and the mixture was stirred. 0.75 mL (5.47 mmol) of WSCD and 555 mg (4.10 mmol) of HOBt·H₂O were added one by one at room temperature, and agitation at room temperature was carried out under argon atmosphere for 69 hours. The solvent was evaporated, 30 mL of pyridine was added and 8.0 mL (84.8 mmol) of acetic anhydride and a catalytic amount of 4-(N,N-dimethylamino)pyridine were added in an ice bath under agitation.

After 140 hours, 10 mL of methanol was added, and chloroform extraction was carried out after 15 minutes. After the organic layer was dried over anhydrous sodium sulfate, the solvent was evaporated and the residual substance (about 6.9 g) was obtained. This residual substance was subjected to column chromatography (200 g of Silica gel N60 (trade name, manufactured by Kanto Chemical Co., Inc.), hexane/ethyl acetate/methanol =12/8/1 → 6/4/1 → 5/5/1), and 859 mg (0.498 mmol, yield: 14.6%) of "Compound 8" was obtained.

859 mg (0.498 mmol) of Compound 8 was dissolved in 20 mL of methanol, and 107 mg of palladium-carbon (act.10%) was added under argon atmosphere. It was stirred under hydrogen atmosphere and subjected to catalytic reduction for about 4 hours. After the reaction ended, the mixture was filtered and the solvent of the filtrate was evaporated. 790 mg of sodium carbonate was added to the residue, and 50 mL of water was added to dissolve the residue. This solution was stirred at room temperature for 22.5 hours, and after adjusted to pH 7 with 1 mol/L of hydrochloric acid, the solvent was evaporated and the residual substance was obtained.

To the residual substance, 5.66 mL of 8 mol/L sodium hydroxide and 5.00 mL of water were added and the mixture was stirred under nitrogen atmosphere, and warmed to 80°C. 3.75 g (10.5 mmol) of anhydrous DTPA was added to this, and the mixture was allowed to react for 30 minutes. Then, the reaction solution was allowed to room temperature, and 8 mol/L sodium hydroxide solution was added to adjust to pH 9, and the mixture was warmed to 80°C again and stirred for 30 minutes. Then, the reaction solution was allowed to room temperature, and concentrated hydrochloric acid was dropped to adjust pH to 1.8, and the mixture was allowed to stand still overnight. The deposited substances were removed by filtration, sodium hydroxide was added to the obtained filtrate, to adjust to pH 8.2, and separation and purification by HPLC was performed on the following conditions.
Column: POROS 50HQ (product name, manufactured by PE biotechnology medical systems, 30 mmID × 240 mm);
Flow rate: 70 mL/min;
Detection: visible-ultraviolet absorptiometer (absorbance: 210 nm);
Elution condition: 150 → 250 mmol/L NaCl/60 min (gradient elution);
Desalination operation was performed on the separated liquid, the solvent was evaporated, and 161 mg (0.11 mmol, yield: 22.0%) of "Compound VII-2" was isolated.
¹H-nmr (500MHz, D₂O, TSP) : 2.62(1H, m, -CH₂CH-), 2.83(2H, m, -COCH₂CH-), 3.52-3.97(54H, m), 4.33(1H, m), 4.72(2H, m).
¹³C-nmr(500MHz, D₂O, TSP) : 33.39, 36. 28 (t), 50. 59 (m), 50. 83 (m), 52. 03 (m), 25. 16 (m), 52. 59 (m), 52. 79 (m), 53. 40, 55.67, 56. 05, 56.68, 57.72, 57.97, 58.08, 58.38, 60.33, 60.92, 61.30, 62.21, 68. 58, 70.15, 71.39, 72.34, 73.57, 74.69, 76.16, 78.55, 79.15, 100.90, 170.90, 171.06, 171.32, 171.48, 174.78, 174.95, 175.09, 175.15, 175. 22.
³¹P-nmr (500MHz, D₂O) : 19. 13, 19.21.
MS (ESI-) : 1467.4328 C₄₉H₈₃N₉O₃₈P₂ requires 1467.4314.

### Example 2 (Labeling of VI-1)

### (1) 111-Indium labeling

A kit was prepared by adjusting the synthesized-compound VI-1 to 1.5 µmol/0.5 mL by adding thereto sodium acetate buffer solution (pH 4.4), and 0.5 mL of indium chloride (¹¹¹InCl₃) was added, and the mixture was allowed to stand at room temperature to perform the labeling reaction. The mixture was analyzed by silica gel TLC using 10% ammonium acetate/methanol as a developing solvent. Consequently, ¹¹¹InCl₃ was not observed and it was confirmed that VI-1 had been labeled with ¹¹¹In.

### (2) 99m-Technetium labeling

The synthesized-compound VI-1 was mixed with a stannous chloride aqueous solution. A physiological saline containing pertechnetate of 17 to 20 mCi was added to the mixture, and the mixture was allowed to stand still at room temperature to perform the labeling reaction.

### Example 3 (Study of in vivo distribution)

The sample was administered via the tail vein under ravonal anesthesia to SD rats (female, 8 to 9-week age, n≥3) under non-fasting. At each time point after administration, blood was collected from the abdominal aorta to allow the animal to die due to the loss of blood, the organs were isolated, radioactivity was counted and organ weights were measured, and the distribution was computed. The measurement results are shown as %ID/g for organ and as %ID for urine.

**Table 1:**

| *In vivo* distribution of 99 mTc-labeled VI-1 | | |
|---|---|---|
| Organs | 1-hour point | 2-hour point |
| Blood | 0.038±0.009 | 0.014±0.002 |
| Lower limb bone | 2.826±0.167 | 2.956±0.114 |
| Liver | 0.024±0.005 | 0.018±0.005 |
| Kidney | 1.281±0.997 | 0.992±0.797 |
| Urine (%ID) | 57.598±7.869 | 62.899±2.590 |

**Table 2:**

| *In vivo* distribution of 99 mTc-MDP injection (comparative and referential example) | |
|---|---|
| Organs | 1-hour point |
| Blood | 0.068±0.012 |
| Lower limb bone | 2.649±1.835 |
| Liver | 1.389±0.296 |
| Kidney | 2.649±1.835 |
| Urine (%ID) | 44.674±2.565 |

As shown above, excretion into urine was rapid and blood clearance was also rapid. As for affinity with calcified tissues, high accumulation was observed. In addition, in consideration of the fact that the composition of the present invention in the above-mentioned example was not purified nor optimized by any means in remarkable contrast to the commercially available composition MDP for injection shown above as a control, it will be apparent that the optimized composition of the present invention will exhibit still more dramatic superiority.

### Example 4 (Selective Boc-substitution reaction in chitotriitol)

Selective Boc-substitution reaction in chitotriitol was carried and derived as shown in the following scheme.

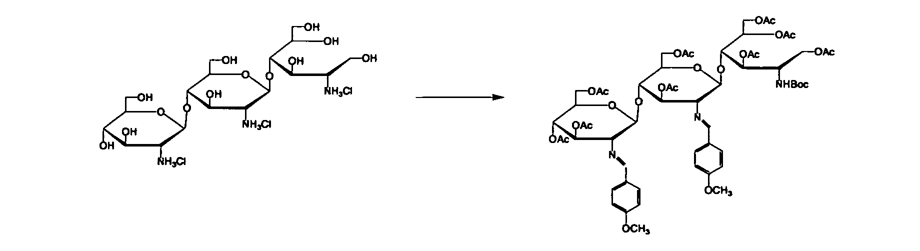

Chitotriitol trichloride (3.06 g, 5.00 mmol) was dissolved in 20 mL of methanol and 40 mL of water, and 2.23 g (21.1 mmol) of sodium carbonate and 1.31 g (6.00 mmol) of dibutyl dicarbonate were added. This reaction mixture was stirred at room temperature for 22 hours, and the solvent was condensed under reduced pressure. The obtained residual substance was dissolved in 50 mL of methanol, 1.63 g (12.0 mmol) of p-anisaldehyde was added, and the mixture was stirred at room temperature for 24 hours. Subsequently, the reaction liquid was concentrated under reduced pressure and after the obtained residual substance was washed with hexane, it was dried under reduced pressure at room temperature overnight. This residual substance was suspended in 30 mL of pyridine, and 12.3 g (120 mmol) of acetic anhydride was dropwised in an ice bath. The reaction liquid was allowed to room temperature, and after 17.5 hour agitation, 10 mL of methanol was added and agitation was performed for 40 more minutes in an ice bath, and then it was poured into an iced water (about 800 mL) under agitation. The deposited precipitate was filtered, dried under reduced pressure at room temperature overnight, and 5.34 g (yield: 87.7%, white powder) of the target compound was obtained.
¹H-nmr (270MHz, CDCl₃, δ) : 1. 40 (s, 9H) , 1. 85 (s, 3H) , 1. 91 (s, 3H) , 1. 92 (s, 3H) , 1. 99 (s, 3H) , 2. 01 (s, 3H) , 2. 03 (s, 3H) , 2. 05 (s, 3H) , 2. 10 (s, 3H) , 2. 11 (s, 3H) , 3. 28 (q, J=8. 6Hz, 2H) , 3. 51 (br d, 1H) , 3. 84 (s, 3H) , 3. 85 (s, 3H) , 3. 88-3. 78 (m, 1H) , 4. 12-3. 96 (m, 4H) , 4. 60-4. 21 (m, 6H) , 4. 82-4. 71 (m, 3H) , 4. 98 (d, J=9. 2H_{Z}, 1H) , 5. 06 (t, J=9. 6Hz, 2H) , 5. 20 (t, J=9. 6Hz, 1H) , 5. 30 (t, J=9. 6Hz, 1H) , 6. 91 (d, J=8. 6Hz, 2H) , 6. 93 (d, J=8.6Hz, 2H) , 7.64 (d, J=8. 6Hz, 2H) , 7.67 (d, J=8. 6Hz, 2H) , 8.07 (s, 1H) , 8. 09 (s, 1H) .

### Industrial Applicability

According to the present invention, a compound in which a group AC having affinity with a calcified tissue is bonded to a mother core MC having a controlled molecular size is provided. This compound shows excellent affinity with a calcified tissue, and the compound which fails to accumulate in the calcified tissues exhibit high excretability in urine. Furthermore, a ligand LI can be bonded to the mother nucleus MC, so that the function of labeling by complex formation or the like is assigned to the ligand LI, the group AC having affinity with calcified a tissue is prevented from forming a complex, and thereby clearance from blood and/or soft tissues and excretion into urine are further promoted.

## Claims

1. A compound having affinity with a calcified tissue represented by the formula: (AC)ₐ-MC-(LI)_{b}
wherein MC is a mother nucleus and represents a residue of a compound having a plurality of functional groups selected from the group consisting of an amino group, an amide group, a hydroxyl group, a thiol group, a thioether group, a sulfonyl group, a phosphonyl group, an aldehyde group, a carboxyl group, a carbonyl group, a halogen, and a cyano group;
AC is a group having affinity with a calcified tissue;
LI is a ligand for binding to a metal atom; and
a is an integer of 1 or more, and b is 0 or an integer of 1 or more.

2. The compound having affinity with a calcified tissue according to claim 1, wherein the mother nucleus MC is a residue of a compound selected from the group consisting of a monosaccharide, an oligosaccharide, an amino oligosaccharide, a cyclodextrin and a saccharide dendrimer.

3. The compound having affinity with a calcified tissue according to claim 1 or 2, wherein the AC is selected from the group consisting of polyaspartic acid, polyglutamic acid and organic phosphonic acid.

4. The compound having affinity with a calcified tissue according to claim 1, wherein the mother nucleus MC is a residue of a compound selected from the group consisting of an oligosaccharide, an amino oligosaccharide, a cyclodextrin and a saccharide dendrimer, and the group AC having affinity with a calcified tissue is bonded to a constituent monosaccharide of the mother nucleus MC, and the ligand LI for binding to a metal atom is bonded to a constituent monosaccharide other than the above-mentioned constituent monosaccharide.

5. The compound having affinity with a calcified tissue according to claim 4, wherein a plurality of the groups AC having affinity with a calcified tissue or a plurality of the ligands LI for binding to a metal atom are bonded to the mother nucleus MC.

6. The compound having affinity with a calcified tissue according to any one of claims 1 to 5, wherein at least one of the mother nucleus MC, the group AC having affinity with a calcified tissue and the ligand LI contains a metal atom or an isotope of a halogen atom, carbon, oxygen, nitrogen, sulfur or phosphorus.

7. The compound having affinity with a calcified tissue according to any one of claims 1 to 6, which forms a complex with a metal atom.

8. The compound having affinity with a calcified tissue according to any one of claims 1 to 7, wherein the mother nucleus MC is a residue of a linear or branched oligosaccharide of 2 to 20 saccharide units which comprises a constituent monosaccharide selected from the group consisting of glucose, mannose and galactose.

9. The compound having affinity with a calcified tissue according to any one of claims 1 to 7, wherein the mother nucleus MC is a residue of a linear or branched amino oligosaccharide of 2 to 20 saccharide units which comprises a constituent monosaccharide selected from the group consisting of glucosamine, mannosamine and galactosamine.

10. The compound having affinity with a calcified tissue according to claim 9, wherein a part of the amino oligosaccharide that constitutes the mother nucleus MC is reduced.

11. The compound having affinity with a calcified tissue according to claim 9, wherein a part of the amino oligosaccharide that constitutes the mother nucleus MC is N-acetylated.

12. The compound having affinity with a calcified tissue according to any one of claims 8 to 11, wherein the oligosaccharide or amino oligosaccharide comprises constituent monosaccharides that are α- or β-linked.

13. The compound having affinity with a calcified tissue according to any one of claims 8 to 11, wherein the oligosaccharide or amino oligosaccharide comprises constituent monosaccharides that are 1-3, 1-4 or 1-6-linked.

14. The compound having affinity with a calcified tissue according to any one of claims 1 to 7, wherein the mother nucleus MC comprises a residue of a cyclodextrin selected from the group consisting of α-, β- and γ-cyclodextrins.

15. The compound having affinity with a calcified tissue according to claim 14, wherein the cyclodextrin is a dialdehyde saccharide which comprises a constituent monosaccharide that is reduced at positions 2 and 3.

16. The compound having affinity with a calcified tissue according to any one of claims 1 to 7, wherein the mother nucleus MC comprises a residue of a saccharide dendrimer, and the saccharide dendrimer comprises a linear or branched saccharide bonded to a core comprising a polycarboxylic acid or an alkyl polycarboxylic acid.

17. The compound having affinity with a calcified tissue according to any one of claims 1 to 7, wherein the mother nucleus MC comprises a residue of a saccharide dendrimer, and the saccharide dendrimer comprises a linear or branched saccharide bonded to a core comprising a polyamine or an alkylpolyamine.

18. The compound having affinity with a calcified tissue according to any one of claims 1 to 17, wherein the group AC having affinity with a calcified tissue comprises an organic phosphonic acid, and the organic phosphonic acid is a residue of a diphosphonic acid represented by the following formula I, derivatives thereof or salts thereof: 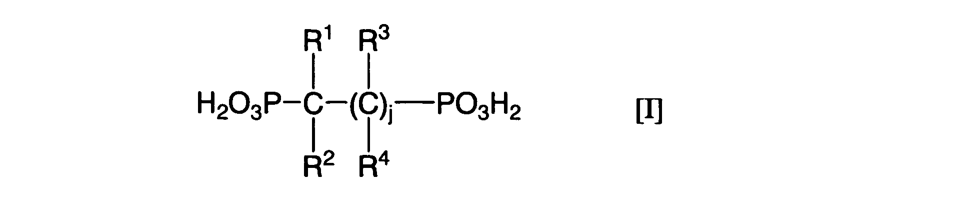 (wherein,
R¹ and R³, which are the same or different, each represents a formula -(CR⁵R⁶)ₖ-R⁷ₗ-(CR⁸R⁹)ₘ-R¹⁰ₙ-(CR¹¹R¹²)ₒ-R¹³ₚ-(CR¹⁴R¹⁵)_{q}R¹⁶ (wherein R⁵, R⁶, R⁸, R⁹, R¹¹, R¹², R¹⁴, R¹⁵ and R¹⁶ are groups each independently selected from the group consisting of H, -OH, -COOH, -C(NH₂)=NH, -CN, -SO₃H, -NR¹⁷₂ and a halogen atom, R¹⁷ is independently H or - (CH₂)ᵣCH₃ respectively, R⁷, R¹⁰ and R¹³ are groups each independently selected from the group consisting of sulfur, oxygen, amide, imide, a divalent heterocycle consisting of 3 to 12 atoms and a cyclic hydrocarbon (Ar(R¹⁸ᵣ-R¹⁹)ₛ), R¹⁸ is -CR⁵R¹⁷, R¹⁹ is independently selected from the group consisting of H, -OH, -COOH, - C(NH₂)=NH, -CN, -SO₃H, -NH₂, -NHMe, -NMe₂ and a halogen atom; k, l, m, n, o, p and q are each independently 0 or an integer of 1 or more, r is 0 to 3, s is 0 to 12, and the sum total of k, l, m, n, o, p and q is 0 to 12);
R² is a group selected from H, -OH, -NH₂, -NHMe, -NMe₂, - CN, and a lower alkyl group (which may be substituted with one or a plurality of polar groups);
R⁴ is a group selected from H, -OH, -NH₂, -NHMe, -NMe₂, - CN, -SO₃H, a halogen and a lower alkyl group (which may be substituted with one or a plurality of polar groups); and
j is 0 or 1 (provided that, when j is 0, R¹ is not H and when j is 1, both of R¹ and R³ cannot be H).

19. The compound having affinity with a calcified tissue according to any one of claims 1 to 17, wherein the group AC having affinity with a calcified tissue comprises an organic phosphonic acid, and the organic phosphonic acid is an organic aminophosphonic acid derivative having an amine nitrogen atom to which a group represented by the formula II is bonded, or an ester or a salt thereof: 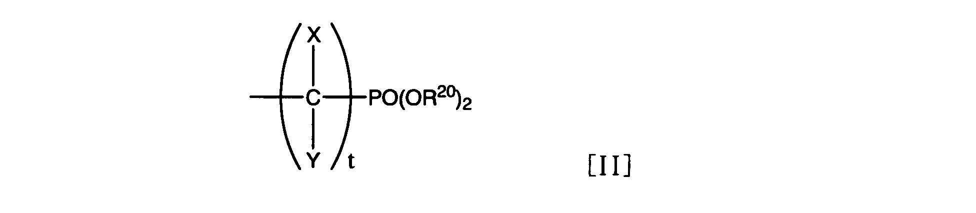 (wherein t is an integer of 1 to 8; X and Y are each independently selected from hydrogen, a halogen group, a hydroxyl group, a carboxyl group, a carbonyl group, a phosphonic acid group, and a hydrocarbon group having 1 to 8 carbon atoms, and when t is larger than 1, each X and Y may be the same or different; R²⁰ is selected from hydrogen, a silyl group, an alkyl group, a benzyl group, sodium and potassium).

20. The compound having affinity with a calcified tissue according to any one of claims 1 to 17, wherein the group AC having affinity with a calcified tissue comprises an organic phosphonic acid, and the organic phosphonic acid is a phosphonic acid derivative represented by the formula III, an ester or a salt thereof. 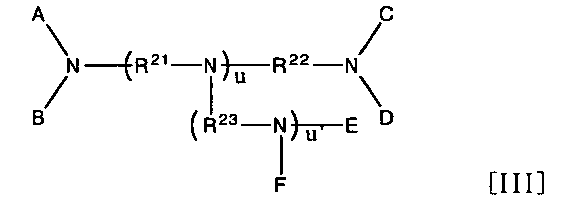 (wherein each u and u' is independently an integer of 0 to 5, preferably 0, 1, or 2; R²¹, R²² and R²³ are each independently -(CH₂)ᵥ- (v= 1 to 5); A, B, C, D, E, and F are each independently selected from the group consisting of hydrogen, a methyl group, an ethyl group, an isopropyl group, a pivaloyl group, a benzyl group, an acetyl group, a trifluoroacetyl group, and groups of the following formulae IV-1 to 3, and one of A, B, C, D, E and F is the group of following formula IV-1. 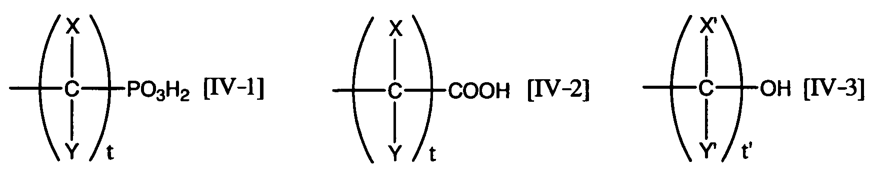 (wherein t, X and Y are the same as in the above-mentioned formula II; t' is 2 or 3; X' and Y' are each independently selected from hydrogen, a methyl group and an ethyl group, and each X' and Y' may be the same or different)).

21. The compound having affinity with a calcified tissue according to any one of claims 1 to 20, wherein the ligand (LI) for binding to a metal atom has a coordinating atom selected from oxygen, sulfur, phosphorus, nitrogen and carbon.

22. The compound having affinity with a calcified tissue according to any one of claims 1 to 20, wherein the ligand (LI) for binding to a metal atom is selected from the group consisting of ethylenediaminetetraacetic acid (EDTA), diethylenetriaminepentaacetic acid (DTPA), triethylenetetraaminehexaacetic acid (TTHA), cyclam, 1,4,8,11-tetraazacyclotetradecane-1,4,8,11-tetraacetic acid (TETA), 1,4,7,10-tetraazacyclododecane-1,4,7,10-tetraacetic acid (DOTA), N{1-2,3-dioleyloxy}propyl}-N,N,N-triethylammonium (DOTMA), mercaptoacetylglycylglycine (MAG3), ethylene cysteine dimer (ECD), hydrazinonicotinyl (HYNIC), lysine-tyrosine-cysteine (KYC), cysteine-glycine-cysteine (CYC), N,N'- bis(mercaptoacetamide)ethylenediamine (DADS), N,N'- bis(mercaptoacetamide)-2,3-diamine propanoic acid (CO2DADS), N,N'-bis(2-mercaptoethyl)ethylenediamine (BATs), thiosemicarbazone, propylene amineoxime (PnAO), and other amineoxime ligands and derivatives thereof.

23. The compound having affinity with a calcified tissue according to any one of claims 1 to 21, wherein the AC or LI has a linker L through which the AC or LI is coupled with the mother nucleus MC.

24. The compound having affinity with a calcified tissue according to claim 22, wherein the linker L is selected from the group consisting of peptide, alkyl, and alkyl ether, alkylamide, alkylamine and alkylolefin represented by formula - (CH₂)_{w}-R²⁴-(CH₂)_{w}- (wherein w is each independently 0 to 5, and R²⁴ is O, S, NHCO, NH, or CH=CH).

25. The compound having affinity with a calcified tissue according to claim 1, which is represented by the following formula V-1 or V-2: 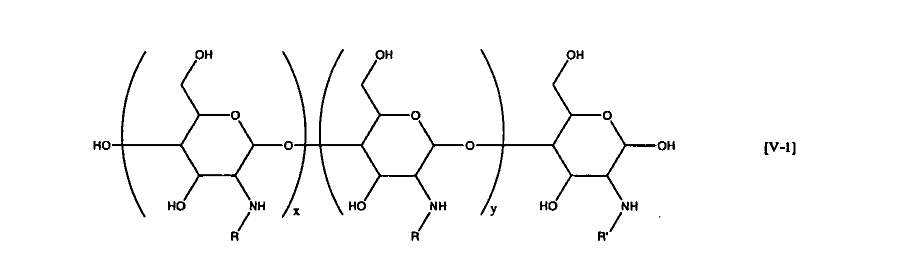 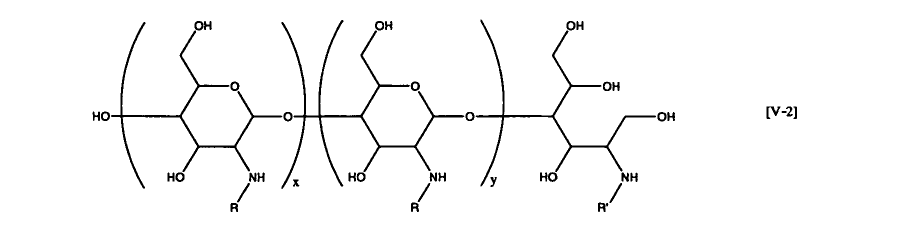 (wherein R and R' are each independently a group AC having affinity with a calcified tissue or a ligand LI for binding to a metal atom, and at least one of them is the group AC having affinity with a calcified tissue; x and y are each independently 0 to 19; and x+y is 1 to 19).

26. The compound having affinity with a calcified tissue, represented by the following formula VI-1:

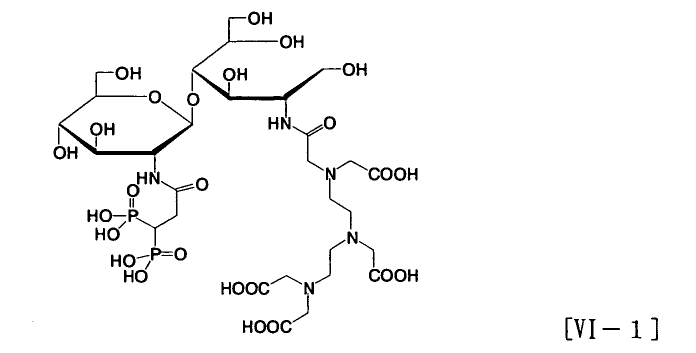

27. The compound having affinity with a calcified tissue, represented by the following formula VI-2:

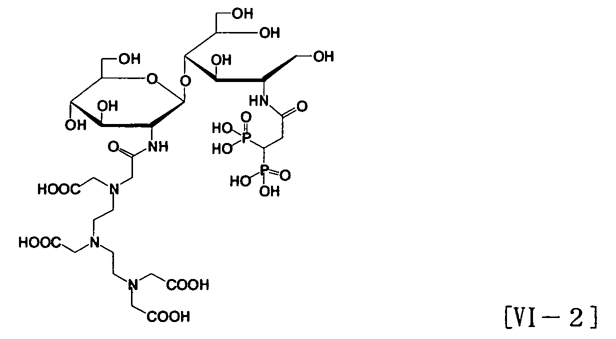

28. The compound having affinity with a calcified tissue, represented by the following formula VII-1:

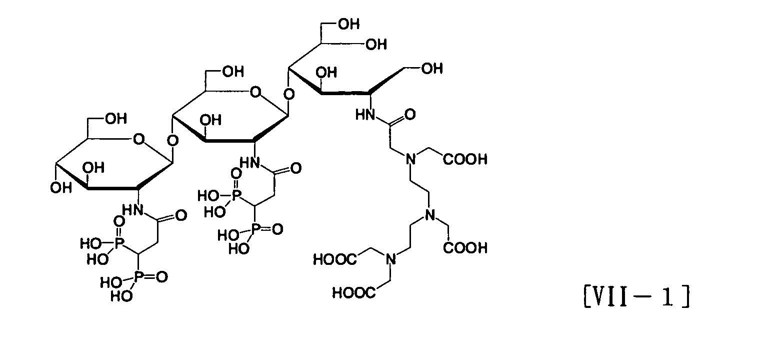

29. The compound having affinity with a calcified tissue, represented by the following formula VII-2:

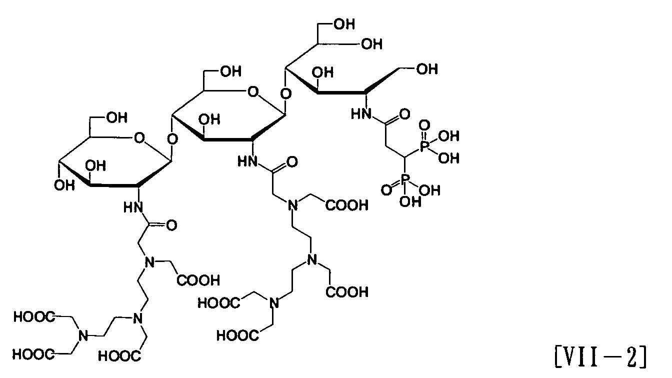

30. The compound having affinity with a calcified tissue according to any one of claims 25 to 29, which forms a complex with a metal atom.

31. The compound having affinity with a calcified tissue according to any one of claims 1 to 30, wherein the metal atom which forms a complex or a metal atom or isotope element contained in the mother nucleus MC, the group AC having affinity with a calcified tissue or the ligand LI is an element selected from the group consisting of elements of atomic number 6-9, 15-17, 21-29, 31, 35, 37-44, 49, 50, 53, 56-70, 72-75, 81, 83 and 85.

32. The compound having affinity with a calcified tissue according to claim 31, wherein the metal atom is radioactive, paramagnetic or X-ray impermeable.

33. The compound having affinity with a calcified tissue according to any one of claims 1 to 30, wherein the metal atom or isotope element is a radioactive nuclide selected from the group consisting of 11-C, 15-O, 18-F, 32-P, 59-Fe, 67-Cu, 67-Ga, 81-Rb, 89-Sr, 90-Y, 99m-Tc, 111-In, 123-I, 124-I, 125-I, 131-I, 117m-Sn, 153-Sm, 186-Re, 188-Re, 201-T1, 211-At, 212-Bi and 213-Bi.

34. The compound having affinity with a calcified tissue according to any one of claims 1 to 30, wherein the metal atom or isotope element is an element selected from the group consisting of chromium (III), manganese (II), iron (II), iron (III), praseodymium (III), neodymium (III), samarium (III), ytterbium (III), gadolinium (III), terbium(III), dysprosium (III), holmium (III), and erbium (III).

35. The compound having affinity with a calcified tissue according to any one of claims 1 to 30, wherein the metal atom or isotope element is an element selected from the group consisting of bismuth, tungsten, tantalum, hafnium, lanthanum, lanthanide, barium, molybdenum, niobium, zirconium and strontium.

36. The compound having affinity with a calcified tissue according to any one of claims 1 to 35, which is in a form of a salt, a hydrate, a solvate, an aggregate, an aqueous solution or a lyophilized product.

37. The compound having affinity with a calcified tissue according to any one of claims 1 to 36, wherein the particle size is 1 nm to 50 µm.

38. A composition for producing a complex compound having affinity with a calcified tissue, which comprises a compound having affinity with a calcified tissue according to any one of claims 1 to 6 and 8 to 29, a peroxide ion of a transition metal, and a reducing agent.

39. A therapeutic agent which comprises a compound having affinity with a calcified tissue according to any one of claims 1 to 37.

40. A pharmaceutical composition which comprises a compound having affinity with a calcified tissue according to any one of claims 1 to 37 or a salt thereof and at least one pharmacologically acceptable carrier.

41. A kit for preparing a radioactive labeled compound, which comprises a compound having affinity with a calcified tissue according to any one of claims 1 to 37.

42. A diagnostic agent, imaging agent or therapeutic agent, which comprises a compound having affinity with a calcified tissue according to any one of claims 1 to 37.

43. A radioactive labeled compound diagnostic agent, imaging agent or therapeutic agent, which comprises a compound having affinity with a calcified tissue according to claim 33, a salt or an aggregate thereof.

44. A nuclear magnetic resonance imaging agent which comprises a compound having affinity with a calcified tissue according to claim 34, a salt or an aggregate thereof.

45. An X-ray imaging agent which comprises a compound having affinity with a calcified tissue according to claim 35, a salt or an aggregate thereof.

46. A method of selectively modifying an amino group at a terminal end, which comprises providing an amino oligosaccharide having 2 to 50 saccharide units which consists of one or more monosaccharides selected from the group consisting of glucosamine, mannosamine and galactosamine and is reduced at a terminal end thereof, and subjecting the amino oligosaccharide to a reaction for generating a carbamate compound.

47. A method of selectively modifying an amino group at a terminal end with a butoxycarbonyl (Boc) group, which comprises reacting, dibutyl dicarbonate, an aminosaccharide of 2 to 13 saccharide units which consists of one or more monosaccharides selected from the group consisting of glucosamine, mannosamine and galactosamine and reduced at a terminal end thereof.
